(19) Europäisches Patentamt
European Patent Office
Office européen des brevets

(11) EP 1 622 940 B1

(12) EUROPÄISCHE PATENTSCHRIFT

(45) Veröffentlichungstag und Bekanntmachung des Hinweises auf die Patenterteilung:
**03.09.2008 Patentblatt 2008/36**

(21) Anmeldenummer: **04727840.3**

(22) Anmeldetag: **16.04.2004**

(51) Int Cl.:
***C07K 14/72*** (2006.01) ***C12Q 1/68*** (2006.01)

(86) Internationale Anmeldenummer:
**PCT/EP2004/004035**

(87) Internationale Veröffentlichungsnummer:
**WO 2004/094470 (04.11.2004 Gazette 2004/45)**

(54) **ANALYSE UND VERWENDUNG VON PAR 1 POLYMORPHISMEN ZUR RISIKOABSCHÄTZUNG FÜR KARDIOVASKULÄRE ERKRANKUNGEN**

ANALYSIS AND USE OF PAR 1 POLYMORPHISMS FOR THE RISK ESTIMATION OF CARDIOVASCULAR DISEASES

ANALYSE ET UTILISATION DE POLYMORPHISMES DE PAR 1 DANS L'EVALUATION DE RISQUES POUR DES MALADIES CARDIOVASCULAIRES

(84) Benannte Vertragsstaaten:
**AT BE BG CH CY CZ DE DK EE ES FI FR GB GR HU IE IT LI LU MC NL PL PT RO SE SI SK TR**

(30) Priorität: **24.04.2003 DE 10318496**

(43) Veröffentlichungstag der Anmeldung:
**08.02.2006 Patentblatt 2006/06**

(73) Patentinhaber: **Sanofi-Aventis Deutschland GmbH**
**65929 Frankfurt am Main (DE)**

(72) Erfinder:
- **KOZIAN, Detlef**
  **65926 Frankfurt am Main (DE)**
- **CZECH, Joerg**
  **35041 Marburg (DE)**
- **SIEGLER, Karl-Ernst**
  **67069 Ludwigshafen (DE)**
- **DELEUZE, Jean-Francois**
  **F-77380 Combs La Ville (FR)**
- **RICARD, Sylvain**
  **F-75013 PARIS (FR)**
- **MACE, Sandrine**
  **F-78350 Jouy-en-Josas (FR)**

(56) Entgegenhaltungen:
- **SCHMIDT V.A. ET L.,: "genomic cloning and characterization of the human thrombin receptor gene" J. BIOLOGICAL CHEMISTRY, Bd. 271, Nr. 16, 19. April 1996 (1996-04-19), Seiten 9307-9312, XP002295968 in der Anmeldung erwähnt**
- **ARNAUD E. ET AL.,: "protective effect of a thrombin receptor (protease-activated receptor 1) gene polymorphism toward venous thromboembolism" ARTERIOSCLER. THROMB. VASC. BIOL., Bd. 20, Februar 2000 (2000-02), Seiten 585-592, XP002295969**
- **PARK H-Y. ET AL.,: "identification of new single-nucleotide polymorphism in the thrombin receptor gene and their effects on coronary artery diseases in koreans" CLIN. EXPER. PHARMACOLOGY AND PHYSIOLOGY, Bd. 27, September 2000 (2000-09), Seiten 690-693, XP002295970**
- **DUPONT A . ET. AL.,: "an intronic polymorphism in the PAR-1 gene associated with the platelet recptor density and the response to SFLLRN" HEMOSTASIS, THROMBOSIS, AND VASCULAR BIOLOGY, Bd. 101, Nr. 5, 1. März 2003 (2003-03-01), Seiten 1833-1840, XP002295971**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 622 940 B1

## Beschreibung

Die Erfindung betrifft Polynukleotidsequenzen umfassend genetische Variationen des PAR1 Gens an den Positionen 3090 und/oder 3329.

Der Protease-aktivierte Rezeptor 1 (PAR1) ist ein Thrombin Rezeptor, der zur Klasse der G Protein-gekoppelten Rezeptoren (GCPR) gehört. Das Gen für PAR1 liegt auf Chromosom 5q13, besteht aus zwei Exonen und deckt eine Region von ca. 27 kb ab. PAR1 wird in unter anderem in Endothelzellen, glatten Muskelzellen, Fibroblasten, Neuronen und humanen Blutplättchen exprimiert. In Blutplättchen ist PAR1 ein wichtiger Rezeptor der Signalübertragung welcher an der Initiation der Aggregation von Blutplättchen beteiligt ist.

Die Aktivierung von PARs erfolgt über die proteolytische Abspaltung eines Teils des N-Terminus der PARs, wodurch eine neue N-terminale Sequenz freigelegt wird, die dann den Rezeptor aktiviert.

Bei der Aktivierung von Blutplättchen spielen PAR1 und PAR4 eine zentrale Rolle; die Aktivierung dieser Rezeptoren führt in Blutplättchen zu morphologischen Veränderungen, Freisetzung von ADP und Aggregation der Blutplättchen.

Ein Zusammenhang von koronaren Herzkrankheiten mit Single Nucleotide Polymorphisms (SNP) in der Promotor-Region von PAR1 in einer koreanischen Patientengruppe konnte nicht bestätigt werden. Ein protektiver Effekt für die Entstehung von venösen Thromboembolien wurde in einer anderen Studie für eine PAR1 Promotorvariante gezeigt.

Die Sequenz des humanen PAR1-Gens ist bekannt. Die Polynukleotidsequenz dieses Gens ist unter der Nummer NM_001992 bei der NCBI Nucleotide-Database abrufbar. Ebenso ist die Proteinsequenz unter der Nummer NP_001983 bei der NCBI Protein Database zugänglich. NCBI ist das National Center for Biotechnology Information (Postadresse: National Center for Biotechnology Information, National Library of Medicine, Building 38A, Bethesda, MD 20894, USA; Web-Adresse:
www.ncbi.nhm.nih.gov). Die Klonierung des PAR1 Gens wurde unter anderem beschrieben in "Schmidt et al., J. Biol. Chem. 271, 9307-9312, 1996".

Neu sind verschiedene Polymorphismen des PAR1 Gens, mittels derer eine stärkere Disposition eines Individuums für koronare Herzkrankheiten festgestellt werden kann. Die betroffenen Individuen erhalten damit die Möglichkeit, durch entsprechende Anpassung in ihrer Lebensführung diesem Risikofaktor rechtzeitig entgegensteuern zu können, in dem beispielsweise durch vermehrte Kontrolle von anderen schädlichen Einflüssen wie Rauchen, Alkoholkonsum, cholesterinreiches Essen, Bluthochdruck und anderem ein Ausgleich geschaffen wird.

Ohne Kenntnis der im weiteren näher erläuterten PAR1 Polymorphismen und deren Verwendung in entsprechenden Verfahren wären solche die Gesundheit betreffenden Vorsorgemechanismen nicht möglich.

Varianten einer bestimmten Nukleotidsequenz mit Austauschen an einzelnen Positionen sind dem Fachmann unter dem Begriff SNP (= Single nucleotide polymorphism) bekannt.

Die Erfindung betrifft eine isoliert vorliegende Polynukleotidsequenz des PAR1 Gens, welche dadurch gekennzeichnet ist, dass an Position 3090 der PAR1 Sequenz gemäß NM_001992, die als Stand der Technik der Öffentlichkeit zur Verfügung steht, ein Austausch des T gegen ein C vorliegt. In einer bevorzugten Ausführungsform umfasst die Polynukleotidsequenz des PAR1 Gens mit einem Austausch von T nach C an Position 3090 eine Sequenz gemäß SEQ ID Nr. 2 und in einer besonders bevorzugten Ausführungsform dieser Polynukleotidsequenz besteht diese aus einer Sequenz der SEQ ID Nr. 2.

Die Erfindung betrifft weiterhin eine isoliert vorliegende Polynukleotidsequenz des PAR1 Gens, welche dadurch gekennzeichnet ist, dass an Position 3329 der PAR1 Sequenz gemäß NM_001992, die als Stand der Technik der Öffentlichkeit zur Verfügung steht, ein Austausch des A gegen ein C vorliegt. In einer bevorzugten Ausführungsform umfasst die Polynukleotidsequenz des PAR1 Gens mit einem Austausch von A nach C an Position 3329 eine Sequenz gemäß SEQ ID Nr. 3 und in einer besonders bevorzugten Ausführungsform dieser Polynukleotidsequenz besteht diese aus einer Sequenz der SEQ ID Nr. 3.

Die Erfindung betrifft auch eine isoliert vorliegende Polynukleotidsequenz des PAR1 Gens, welche dadurch gekennzeichnet ist, dass an Position 3090 der PAR1 Sequenz gemäß NM_001992 ein Austausch des T gegen ein C und gleichzeitig an Position 3329 dieser PAR1 Sequenz ein Austausch des A gegen ein V vorliegt. In einer bevorzugten Ausführungsform umfasst die Polynukleotidsequenz des PAR1 Gens mit einem Austausch von T nach C an Position 3090 und einen gleichzeitig vorliegenden Austausch von A nach C an Position 3329 eine Sequenz gemäß SEQ ID Nr. 4 und in einer besonders bevorzugten Ausführungsform dieser Polynukleotidsequenz besteht diese aus einer Sequenz der SEQ ID Nr. 4.

Die Erfindung bezieht sich auch auf einen isoliert vorliegenden Teil der Polynukleotidsequenz des PAR1 Gens bestehend aus einer Sequenz gemäß SEQ ID Nr. 5.

Die Erfindung bezieht sich auf einen isoliert vorliegenden Teil der Polynukleotidsequenz des PAR1 Gens, welche sich dadurch auszeichnet, dass an Position 3090 bezogen auf die PAR1 Sequenz gemäß NM_001992 ein Austausch des T gegen ein C vorliegt, bestehend aus einer Sequenz gemäß SEQ ID Nr. 6.

Die Erfindung bezieht sich außerdem auf einen isoliert vorliegenden Teil der Polynukleotidsequenz des PAR1 Gens, welche sich dadurch auszeichnet, dass an Position 3329 bezogen auf die PAR1 Sequenz gemäß NM_001992 ein Austausch des A gegen ein C vorliegt bestehend aus einer Sequenz gemäß SEQ ID Nr. 7. Die Erfindung bezieht sich auch auf einen isoliert vorliegenden Teil der Polynukleotidsequenz des PAR1 Gens, welche sich dadurch auszeichnet, dass an Position 3090 bezogen auf die PAR1 Sequenz gemäß NM_001992 ein Austausch des T gegen ein C und gleichzeitig an Position 3329 der genannten PAR1 Sequenz ein Austausch des A gegen ein C vorliegt bestehend aus einer Sequenz gemäß SEQ ID Nr. 8.

Die Erfindung umfasst weiterhin die Herstellung einer Polynukleotidsequenz des PAR1 cDNA Gens mit einer Länge von 3592 Basenpaaren, welche die vorstehend gekennzeichneten Polymorphismen an den Positionen 3090 und 3329 einzeln oder in Kombination enthalten oder nicht enthalten kann, gekennzeichnet durch die folgenden Verfahrensschritte,

a] Bereitstellung von humaner DNA enthaltend eine PAR1 Sequenz gemäß SEQ ID Nr. 2 und/oder eine PAR1 Sequenz gemäß SEQ ID Nr. 3 und/oder eine PAR1 Sequenz gemäß SEQ ID Nr. 4,
b] Bereitstellung eines Primerpaares mit einer Sequenz gemäß SEQ ID Nr. 9 und SEQ ID Nr. 10,
c] Amplifizierung der PAR1 Polynukleotidsequenz durch die Polymerasekettenverlängerungsreaktion (PCR),
d] Isolierung und/oder Reinigung des entstehenden Fragments mit einer Länge von 3,56 kb aus c]
e] Sequenzzierung des Fragments aus d].

Die Erfindung bezieht sich außerdem auf die Herstellung einer Polynukleotidsequenz gemäß SEQ ID Nr. 5, SEQ ID Nr. 6, SEQ ID Nr. 7 oder SEQ ID Nr. 8, gekennzeichnet durch die folgenden Verfahrensschritte,

a] Bereitstellung von humaner chromosomaler DNA enthaltend eine PAR1 Sequenz gemäß SEQ ID Nr. 1 und/oder eine PAR1 Sequenz gemäß SEQ ID Nr. 2 und/oder eine PAR1 Sequenz gemäß SEQ ID Nr. 3 und/oder eine PAR1 Sequenz gemäß SEQ ID Nr. 4"
b] Bereitstellung eines Primerpaares gemäß SEQ ID Nr. 11 und SEQ ID Nr. 12,
c] Amplifizierung des Fragments der PAR1 Polynukleotidsequenz durch die Polymerasekettenverlängerungsreaktion (PCR),
d] Isolierung und/oder Reinigung des entstandenen Fragments aus c]
e] Sequenzierung des Fragments aus d].

Die Erfindung bezieht sich weiterhin auf ein Verfahren zum Nachweis, ob in einem PAR1 Gen an Position 3090 der Sequenz gemäß NM_001992 ein Austausch von T nach C und/oder an Position 3329 der Sequenz gemäß NM_001992 ein Austausch von A nach C vorliegt oder nicht vorliegt, umfassend die folgenden Verfahrensschritte:

a] Bereitstellung von biologischem Material enthaltend Zellen eines Menschen,
b] Gewinnung von chromosomaler DNA aus dem Material von a],
c] Amplifizierung eines Polynukleotidfragments mittels der Primer gemäß SEQ ID Nr. 11 und SEQ ID Nr. 12 durch eine PCR-Reaktion,
d] Sequenzierung des Polynukleotidfragments aus c].

Weiterhin umfasst die Erfindung ein Verfahren zum Nachweis, ob in einem PAR1 Gen an Position 3090 der Sequenz gemäß NM_001992 ein Austausch von T nach C und/oder an Position 3329 der Sequenz gemäß NM_001992 ein Austausch von A nach C vorliegt oder nicht vorliegt, umfassend die folgenden Verfahrensschritte:

a] Bereitstellung von biologischem Material enthaltend Zellen eines Menschen,
b] Gewinnung von RNA aus dem Material von a],
c] Umschreiben der RNA in cDNA mittels reverser Transkriptase,
d] eventuell Amplifizierung eines Polynukleotidfragments mittels der Primer gemäß SEQ ID Nr. 10 und SEQ ID Nr. 11 durch die PCR-Reaktion,
e] Sequenzierung der cDNA aus c] und/oder des Polynukleotidfragments aus d].

Die Erfindung bezieht sich auch auf ein Verfahren zum Nachweis, ob in einem PAR1 Gen an Position 3090 der Sequenz gemäß NM_001992 ein Austausch von T nach C und/oder an Position 3329 der Sequenz gemäß NM_001992 ein Austausch von A nach C vorliegt oder nicht vorliegt, umfassend die folgenden Verfahrensschritte:

a] Bereitstellung von biologischem Material enthaltend Zellen eines Menschen,
b] Gewinnung von chromosomaler DNA aus dem Material von a],
c] Southern Blotting der chromosomalen DNA aus b],
d] Bereitstellung einer Sonde gemäß SEQ ID Nr. 5 und/oder SEQ ID Nr. 6 und/oder SEQ ID Nr. 7 und/oder SEQ ID Nr. 8,
e] Hybridisierung des Southern Blots aus c] mit der Sonde aus d] unter stringenten Hybridisierungsbedingungen,
f] Bestimmung des Vorliegens oder Nichtvorliegens einer genetischen Variation im Gen PAR1 an Position 3090 und/oder 3329 gemäß NM_001992 durch Vergleich der Ergebnisse der Hybridisierung aus e].

Die Erfindung bezieht sich weiterhin auf ein Verfahren zum Nachweis, ob in einem PAR1 Gen an Position 3090 der Sequenz gemäß NM_001992 ein Austausch von T nach C und/oder an Position 3329 der Sequenz gemäß NM_001992 ein Austausch von A nach C vorliegt oder nicht vorliegt, umfassend die folgenden Verfahrensschritte,

a] Bereitstellung von biologischem Material enthaltend Zellen eines Menschen,
b] Gewinnung von RNA aus dem Material von a],
c] Northern-Blotting der RNA aus b],
d] Bereitstellung einer Sonde gemäß SEQ ID Nr. 5 und/oder SEQ ID Nr. 6 und/oder SEQ ID Nr. 7 und/oder SEQ ID Nr. 8,
e] Hybridisierung des Northern Blots aus c] mit der Sonde aus d] unter stringenten Hybridisierungsbedingungen,
f] Bestimmung des Vorliegens oder Nichtvorliegens einer genetischen Variation im Gen PAR1 an Position 3090 und/oder 3329 gemäß NM_001992 durch Vergleich der Ergebnisse der Hybridisierung.

Der Nachweis der genetischen Variationen oder Polymorphismen im Gen PAR1 an den Positionen 3090 und/oder 3329 kann verwendet werden als (a) genetischer Marker zur Risikoabschätzung des Auftretens von Vorhofflimmern, akutem Koronarsyndrom, Kardiomyopathie und/oder instabiler Angina, als (b) Marker für eine präventive Behandlung von Vorhofflimmern, akutem Koronarsyndrom, Kardiomyopathie und/oder stabiler Angina der Träger der entsprechenden genetischen Varianten, als (c) Marker zur Anpassung der zu verabreichenden Dosis einer pharmazeutisch wirksamen Substanz gegen Vorhofflimmern, akutem Koronarsyndrom, Kardiomyopathie und/oder instabiler Angina, als (d) Marker zur Bestimmung der Hochdurchsatz-Screening Strategie zur Identifizierung einer pharmazeutisch wirksamen Substanz gegen Vorhofflimmern, akutem Koronarsyndrom, Kardiomyopathie und/oder instabiler Angina, als (e) Marker zur Identifizierung der relevanten Individuen bzw. Patienten für klinische Studien um die Verträglichkeit, Sicherheit und Wirksamkeit einer pharmazeutischen Substanz gegen Vorhofflimmern, akutem Koronarsyndrom, Kardiomyopathie und/oder instabiler Angina zu überprüfen und als (f) Basis zur Entwicklung von Testsystemen zur Analyse der genetischen Variation im Gen PAR1 auf DNA, RNA oder Protein Ebene.

Die Erfindung bezieht sich auch auf eine isolierte Polynukleotidsequenz mit einer Anzahl von 21 bis 50 Nukleotiden gekennzeichnet dadurch, dass darin eine Sequenz entsprechend der SEQ ID Nr. 11 enthalten ist. Diese Sequenz besteht bevorzugt aus der SEQ ID Nr. 11. Die Erfindung bezieht sich weiterhin auf eine isolierte Polynukleotidsequenz mit einer Anzahl von 20 bis 50 Nukleotiden **gekennzeichnet dadurch, dass** eine Sequenz entsprechend der SEQ ID Nr. 12 enthalten ist. Diese Sequenz besteht bevorzugt aus der SEQ ID Nr. 12.

Die Erfindung bezieht sich auch auf die Verwendung einer isolierten Polynukleotidsequenz mit einer Länge von 21 bis 50 Nukleotiden, welche eine Sequenz gemäß SEQ ID Nr. 11 umfasst oder aus dieser besteht, in Kombination mit einer isolierten Polynukleotidsequenz mit einer Länge von 20 bis 50 Nukleotiden, welche eine Sequenz gemäß SEQ ID Nr. 12 umfasst oder aus dieser besteht, zur Vermehrung eines entsprechenden Fragments aus dem PAR1 Gen mittels der Polymerasekettenverlängerungsreaktion (PCR). Diese Verwendung betrifft bevorzugt die Vermehrung eines Fragments aus einem PAR1 Gen, welches an Position 3090 der Sequenz gemäß NM_001992 einen Austausch von T nach C aufweist und/oder an Position 3329 der Sequenz gemäß NM_001992 einen Austausch von A nach C aufweist.

Die Erfindung umfasst außerdem einen Kit, welcher enthält

a] eine isolierte Polynukleotidsequenz mit einer Länge von 21 bis 50 Nukleotiden, welche eine Sequenz gemäß SEQ ID Nr. 11 umfasst oder aus dieser besteht,
b] eine isolierte Polynukleotidsequenz mit einer Länge von 20 bis 50 Nukleotiden, welche eine Sequenz geäß SEQ ID Nr. 12 umfasst oder aus dieser besteht,
c] wenigstens ein Enzym zur Durchführung der Polymerasekettenverlängerungsreaktion (PCR),
d] eventuell Substanzen und/oder Lösungen für die Ausführung der Polymerasekettenverlängerungsreaktion,
e] eventuell Polynukleotidsequenzen umfassend das PAR1 Gen mit oder ohne Austausch an Position 3090 der PAR1 Sequenz gemäß NM_001992 und/oder Position 3329 gemäß NM_001992 jeweils in der vollen Länge und/oder Teilen davon
f] sowie eventuell Reagenzien zur Durchführung einer Sequenzzierung.

Als Kit wird hierbei und im folgenden die Kombination der genannten Bestandteile verstanden, die als räumliches Nebeneinander zu einer Funktionseinheit zusammengeschlossen sind.

Die Erfindung bezieht sich weiterhin auf die Herstellung des vorstehend beschriebenen Kit, wobei

a] eine isolierte Polynukleotidsequenz mit einer Länge von 21 bis 50 Nukleotiden, welche eine Sequenz gemäß SEQ ID Nr. 11 umfasst oder aus dieser besteht, bereitgestellt wird,
b] eine isolierte Polynukleotidsequenz mit einer Länge von 20 bis 50 Nukleotiden, welche eine Sequenz gemäß SEQ ID Nr. 12 umfasst oder aus dieser besteht, bereitgestellt wird,
c] ein Enzym für die Durchführung der Polymerasekettenverlängerungsreaktion (PCR) bereitgestellt wird,
d] gegebenenfalls Reagenzien zur Durchführung einer Sequenzzierung bereitgestellt werden
e] eventuell Substanzen und/oder Lösungen für die Ausführung der Polymerasekettenverlängerungsreaktion (PCR) bereitgestellt werden,
f] eventuell Polynukleotidsequenzen umfassend das PAR1 Gen mit oder ohne Austausch an Position 3090 der PAR1 Sequenz gemäß NM_001992 von T nach C und/oder Position 3329 von A nach C gemäß NM_001992 jeweils in der vollen Länge oder Teilen davon bereitgestellt werden,
g] die Komponenten aus a] bis f] jeweils separat in geeignete Behältnisse gegeben werden,
h] die Behältnisse aus g] gegebenenfalls kombiniert in einer oder mehrerer Verpackungseinheiten zusammengefasst werden.

Vorstehend beschriebener Kit kann zur Vermehrung eines Fragments aus dem PAR1 Gen verwendet werden.

In den folgenden Ausführungen werden die technischen Aspekte der Erfindung ausführlicher erörtert.

Isoliert vorliegende Polynukleotidsequenzen des PAR1 Gens können beispielsweise durch Vermehrung mittels der Polymerasekettenverlängerungsreaktion (PCR) hergestellt werden. Geeignete Primer hierfür sind in der SEQ ID Nr. 9 und SEQ ID Nr. 10 beschrieben.
Die PCR ist eine in vitro-Technik, mit der man gezielt Polynukleotid-Abschnitte, die von zwei bekannten Sequenzen eingerahmt werden, vervielfältigen kann. Zur Amplifizierung benötigt man kurze, einzelsträngige DNA-Moleküle, die komplementär zu den Enden einer definierten Sequenz einer DNA- oder RNA Matrize sind (Primer). Eine DNA-Polymerase verlängert unter den richtigen Reaktionsbedingungen und in Gegenwart von Desoxynukleotidtriphosphaten (dNTPs) die Primer entlang der einzelsträngigen und denaturierten Polynukleotidmatrize und synthetisiert so neue DNA-Stränge, deren Sequenz komplementär zur Matrize ist. Dabei wechselt die Temperatur periodisch, so dass die Polynukleotidstränge immer wieder denaturiert werden und sich die Primer anlagern und verlängert werden können. Man verwendet hitzestabile DNA-Polymerasen z.B. die Taq-Polymerase. Ein typischer PCR Ansatz enthält neben einer Polynukleotid Matrize zwei geeignete Primernukleotide beispielsweise in Konzentration zwischen 0,2 bis 2$\mu$M, weiterhin dNTPs beispielsweise mit Konzentrationen von 200 $\mu$M pro dNTP, weiterhin $MgCl_2$ mit einer Konzentration von 1-2mM und 1-10 Einheiten einer hitzestabilen DNA-Polymerase wie beispielsweise der Taq-Polymerase (Polymerase aus Thermus aquaticus). Hitzestabil DNA-Polymerase sowie die Bestandteile zu deren Durchführung samt Protokollen werden von zahlreichen Firmen wie beispielsweise Roche Diagnostics, Clontech, Life Technologie, New England Biolabs, Promega, Stratagene und anderen kommerziell angeboten.

Die Polynukleotid-Matrize zur Vermehrung der zu isolierenden Polynukleotidsequenz kann als RNA oder DNA vorliegen. Wenn die Polynukleotid-Matrize als RNA vorliegt, wird sie vor der eigentlichen PCR Reaktion mittels reverser Transkriptase in DNA umgeschrieben. Die Menge der Polynukleotid-Matrize bei der Ausführung der PCR-Reaktion kann beispielsweise 0,01 bis 20 ng betragen.
Die Gewinnung der Polynukleotid-Matrize erfolgt durch dem Fachmann bekannte Techniken zur Gewinnung von DNA und/oder RNA aus biologischem Material. Biologisches Material soll hierbei unter anderem umfassen die Zellen eines Gewebes oder Organs (beispielsweise Gehirn, Blut, Leber, Milz, Niere, Herz, Blutgefäße) eines Wirbeltiers einschließlich des Menschen, oder Zellen aus einer eukaryotischen Zellkultur (beispielsweise Hela-Zellen, CHO-Zellen, 3T3-Zellen) oder Zellen bestehend aus Bakterien oder Hefen, in denen die zu isolierende DNA-Sequenz in klonierter Form vorliegt.

Zellen eines Gewebeverbandes oder Organs eines Wirbeltiers einschließlich des Menschen können durch Blutentnahme, Gewebepunktion oder operative Techniken gewonnen werden. Eine Polynukleotid-Matrize hieraus erhält man beispielsweise durch Aufschluss der Zellen, eventuell Anreicherung einzelner Organellen insbesondere des Zellkerns und Gewinnung der DNA oder RNA durch Fällung und Zentrifugation.

Eine weitere Methode zur Herstellung von isoliert vorliegenden Polynukleotidsequenzen des PAR1 Gens besteht in der Klonierung des PAR1 Gens, der anschließenden Expression in Bakterien oder Hefen und der Reinigung des exprimierten Polynukleotids. Zur Herstellung eines Polynukleotidfragments, welches kloniert werden kann, eignet sich beispielsweise die zuvor besprochene PCR-Reaktion. Vorteilhafterweise werden bei einem zu klonierenden Fragment Primer verwendet, welche in 5'-Position von der komplementären Sequenz die Erkennungssequenz eines Reaktionsenzyms tragen. Für die beiden Primer können jeweils dieselbe oder verschiedene Erkennungssequenzen für Restriktionsenzyme verwendet werden.
Gebräuchliche Restriktionsenzyme sind beispielsweise: BamH1 (GGATCC), Clal (ATCGAT), EcoR1 (GAATTC), EcoRV (GATATC), HindIII (AAGCTT), Ncol (CCATGG), Sall (GTCGAC), Xbal (TCTAG1).

Zur Klonierung wird ein Vektor mit den Restriktionsenzymen, die den an die Primer angefügten Erkennungssequenzen entsprechen, behandelt. Das Fragment wird durch Isolierung und Behandlung durch dieselben Restriktionsenzyme mittels Ligase mit dem Vektor verbunden. Als Vektor soll ein DNA Molekül, wie etwa ein Plasmid, Bakteriophage oder ein Cosmid verstanden werden, mit dessen Hilfe man Gene oder andere DNA-Sequenzen klonieren und zur Vervielfältigung in eine bakterielle oder eukaryotische Zelle einschleusen kann. Vektoren sind beispielsweise DNA-Moleküle wie pBR322, pUC18/19, pBluescript, pcDNA3.1. Vektoren sind von Spezialfirmen für biotechnisches Material wie Roche Diagnostics, New England Biolabs, Promega, Stratagene u.a. kommerziell zu erhalten.

Der Fachmann findet die zur Durchführung der PCR-Reaktion, der Bereitstellung von Polynukleotiden oder zur Ausführung von Klonierungsarbeiten erforderlichen Handlungsanweisungen in Form von Rezepten und Protokollen in Standardhandbüchern wie beispielsweise in a] "Current Protocols in Molecular Biology by Frederick M. Ausubel (Editor), Roger Brent (Editor), Robert E. Kingston (Editor), David D. Moore (Editor), J. G. Seidman (Editor), Kevin Struhl (Editor), loose leaf edition, continuously updated, John Wiley & Sons, Inc., New York oder in b] Short Protocols in Molecular Biology, 5th edition, by Frederick M. Ansubel (Editor), Roger Brent (Editor), Robert E. Kingston (Editor), David D. Moore (Editor), J.G. Seidman (Editor), John A. Smith (Editor), Kevin Struhl (Editor), October 2002, John Wiley & Sons, Inc., New York" oder in c] "Molecular Cloning by J. Sambrock, E.F. Fritsch, T. Maniatis; Cold Spring Harbor Laboratory Press".

Die Bereitstellung geeigneter Primersequenzen erfolgt beispielsweise über deren chemische Synthese, die auftragsmäßig auf kommerzieller Basis von Firmen wie MWG Biotech und anderen vorgenommen werden kann.

Humane cDNA aus unterschiedlichen Organen ist kommerziell von Firmen wie beispielsweise Promega, Stratagene oder anderen erhältlich.

Die Sequenzierung eines Polynukleotids erfolgt mittels dem Fachmann bekannter Routinemethoden über beispielsweise Laborroboter von Firmen wie beispielsweise Life Technologies, Applied Biosystems, BioRad oder anderen.

Isolierte Polynukleotidsequenzen der PAR1 Variante sowie Fragmente daraus können auch zur Hybridisierung bei unterschiedlicher Stringenz verwendet werden. Stringenz beschreibt Reaktionsbedingungen, die die Spezifität der Hybridisierung oder Anlagerung zweier einzelsträngiger Nukleinsäuremoleküle beeinflussen. Die Stringenz und damit auch die Spezifität einer Reaktion lässt sich steigern, indem man die Temperatur erhöht und die lonenstärke erniedrigt. Niedrige stringente Bedingungen liegen beispielsweise vor, wenn die Hybridisierung bei Raumtemperatur in 2xSSC-Lösung durchgeführt wird. Hoch stringente Bedingungen sind beispielsweise gegeben, wenn bei 68°C in 0,1 x SSC/0,1 % SDS-Lösung hybridisiert wird.

Hybridisierung unter stringenten Hybridisierungsbedingungen im Sinne dieser Anmeldung bedeutet:

1] Hybridisierung der markierten Sonde mit der zu untersuchenden Probe bei 65°C (oder im Falle von Oligonukleotiden 5°C unter der Schmelztemperatur) über Nacht in 50 mM Tris pH 7,5, 1NaCl, 1 % SDS, 10 % Dextran Sulfat, 0,5 mg/ml denaturierte Salmon Sperm DNA.
2] Waschen für 10 Min. bei Raumtemperatur in 2 x SSC.
3] Waschen für 30 Min. bei 65°C (oder im Falle von Oligonukleotiden, 5°C unter der Schmelztemperatur) in 1 x SSC /1 % SDS.
4] Waschen für 30 Min. bei 65°C (oder im Falle von Oligonukleotiden 5°C unter der Schmelztemperatur) in 0,2 x SSC / 0,1 % SDS.
5] Waschen für 30 Min. bei 65°C (oder im Falle von Oligonukleotiden 5°C unter der Schmelztemperatur) in 0,1 % SSC / 0,1 % SDS.

Als Oligonukleotide sollen hierfür DNA-Fragmente mit einer Gesamtlänge von 20 Nukleotiden angesehen werden. Die Schmelztemperatur ergibt sich aus der Formel

$$Tm = 2 \cdot (\text{Anzahl A+T}) + 4 (\text{Anzahl G+C})C°.$$

Zur Herstellung einer 2 x SSC bzw. 0,1 x SSC-Lösung wird eine 20 x SSC-Lösung entsprechend verdünnt. Die 20 x SSC-Lösung besteht aus einer 3M NaCl/0,3 Na Citrat 2 $H_2O$-Lösung. SDS steht für Na-dodecylsulfat.

Für die Durchführung der Hybridisierung werden die zu untersuchenden Polynukleotide nach elektrophoretischer Auftrennung und anschließender Denaturierung auf eine Nylon- oder Nitrozellulosemembran übertragen (Southern Blot - DNA; Northern Blot-RNA). Die Hybridisierung erfolgt mit einer Sonde, welche radioaktiv oder auf andere Weise beispielsweise mit Hilfe von Fluoreszenzfarbstoffen markiert ist. Die Sonde besteht aus einer gewöhnlich einzelsträngigen und/oder denaturierten DNA- oder RNA Polynukleotidsequenz, welche an die komplementäre Nukleotidsequenz der wiederum einzelsträngigen und/oder denaturierten zu untersuchenden DNA- oder RNA Polynukleotidsequenz bindet.

Der Nachweis von Single Nucleotide Polymorphisms des PAR1 Gens kann mit Hilfe der erfindungsgemäßen Primer, auch durch eine SSCP-Analyse vorgenommen werden. SSCP steht für Single Stranded Conformation Polymorphism. Es handelt sich hierbei um eine Elektrophoresetechnik zur Identifizierung einzelner Basenpaaraustausche. Die zu untersuchenden Polynukleotide werden mittels markierter Primer durch die PCR-Reaktion amplifiziert und nach Denaturierung in Einzelstränge in einer Polyacrylamidgelelektrophorese (PAGE) aufgetrennt. Weisen die zu untersuchenden DNA-Fragmente einzelne Basenpaaraustausche auf, so besitzen sie unterschiedliche Konformation und wandern folglich in der PAGE verschieden schnell.

Substanzen zur Durchführung der PCR-Reaktion sind beispielsweise Puffer wie Hepes oder Tris, weiterhin dAPP, dGTP, dTTP, dCTP, sowie $Mg^{2+}$ und eventuell weitere zweiwertige oder einwertige Ionen. Lösungen enthalten diese Substanzen in gelöster Form.

Beispiele

Amplifizierung genomischer Regionen des PAR1 Gens

Zum Nachweis des Nukleotidaustausches T zu C an der Position 3090 und A zu C an der Position 3329 in der PAR1 Sequenz wurden folgende Primer verwendet:

Primer 1: 5'-ACAGAGTGGAATAAGACAGAG-3' (SEQ ID Nr. 11)
Primer 2: 5'-CCAGTGCTAGCTTCTACTTAC-3 (SEQ ID Nr. 12)
Primer 1 (Seq ID Nr. 11) entspricht den Positionen 2767 bis 2789 der NM_001992 Referenzsequenz. Primer 2 stammt aus dem Exon Nr. 1 des PAR1 Gens.

PCR Protokoll zur Amplifizierung:

Verwendete Reagenzien sind von Applied Biosystems (Foster City, USA): 20 ng genomischer DNA; 1 Einheit TaqGold DNA Polymerase; 1 x Taq Polymerase Puffer; 500 μM dNTPs; 2,5 mM MgCl2; 200 nM eines jeden Amplifikations Primer Paares; $H_2O$ ad 5 μl.

Amplifikations Programm der PCR zur Genotypisierung:

| | |
|---|---|
| 95°C für 10 min | x 1 Zyklus |
| 95°C für 30 sec | |
| 70°C für 30 sec | x 2 Zyklen; |
| 95°C für 30 sec | |
| 65°C für 30 sec | x 2 Zyklen; |
| 95°C für 30 sec | |
| 60°C für 30 sec | x 2 Zyklen; |
| 95°C für 30 sec | |
| 56°C für 30 sec | |
| 72°C für 30 sec | x 40 Zyklen; |
| 72°C für 10 min | |
| 4°C für 30 sec | x 1 Zyklus; |

Identifizierung der SNPs

Protokoll zur Minisequenzierung und Nachweis der SNPs.

Alle Reagenzien sind von Applied Biosystems (Foster City, USA). 2 μl gereinigtes PCR Produkt, 1,5 μl BigDye-Terminator-Kit, 200 nM eines Sequenzierungsprimers; $H_2O$ ad 10 μl.

Amplifikationsprogramm zur Sequenzierung:

| | |
|---|---|
| 96°C für 2 min | x 1 Zyklus; |
| 96°C für 10 sec | |

(fortgesetzt)

| | |
|---|---|
| 55°C für 10 sec | |
| 65°C für 4 min | x 30 Zyklen; |
| 72°C für 7 min | |
| 4°C für 30 sec | x 1 Zyklus; |

Analyse der Sequenzierungsprodukte:

Die Sequenzen wurden zunächst mit der Sequenz Analyse Software (Applied Biosystems, Foster City, USA) zum Erhalt der Rohdaten analysiert, dann prozessiert mit Phred, Phrap, Polyphred und Consed. Phred, Phrap, Polyphred und Consed sind Software, geschrieben von Phil Green an der Washington University (http://www.genome.washington.edu).

Zuordnung von PAR1 SNPs zu koronaren Erkrankungen

In einer klinischen Untersuchung wurden zwei PAR1 Polymorphismen aus dem 3'-nicht-kodierenden Bereich des Gens auf einen Zusammenhang mit thrombotischen und kardiovaskulären Komplikationen in einer Patientenkohorte untersucht.

Im weiteren werden die folgenden Abkürzungen benutzt (alle angegebenen Positionen beziehen sich auf die Positionen der Nukleotide in der Referenzsequenz NM_001992).

PAR1 T3090T beschreibt die Gruppe von Personen, bei denen auf beiden Allelen des Gens PAR1 an der Position 3090 ein Thymidin (T) vorhanden ist. Diese Personen sind homozygot bezüglich dieser PAR1 Variante.
PAR1 T3090C beschreibt die Gruppe von Personen, bei denen auf einem Allel des Gens PAR1 an der Position 3090 ein Cytidin (C) vorhanden ist, auf dem anderen Allel des Gens PAR1 an der Position 3090 ein Thymidin (T). Diese Personen sind heterozygot bezüglich dieser PAR1 Variante.
PAR1 C3090C beschreibt die Gruppe von Personen, bei denen auf beiden Allelen des Gens PAR1 an der Position 3090 ein Cytidin (C) vorhanden ist. Diese Personen sind homozygot bezüglich dieser PAR1 Variante.
PAR1 A3329A beschreibt die Gruppe von Personen, bei denen auf beiden Allelen des Gens PAR1 an der Position 3329 ein Adenosin (A) vorhanden ist. Diese Personen sind homozygot bezüglich dieser PAR1 Variante.

PAR1 A3329C beschreibt die Gruppe von Personen, bei denen auf einem Allel des Gens PAR1 an der Position 3329 ein Cytidin (C) vorhanden ist, auf dem anderen Allel des Gens PAR1 an der Position 3329 ein Adenosin (A). Diese Personen sind heterozygot bezüglich dieser PAR1 Variante.
PAR1 C3329C beschreibt die Gruppe von Personen, bei denen auf beiden Allelen des Gens PAR1 an der Position 3329 ein Cytidin (C) vorhanden ist. Diese Personen sind homozygot bezüglich dieser PAR1 Variante.

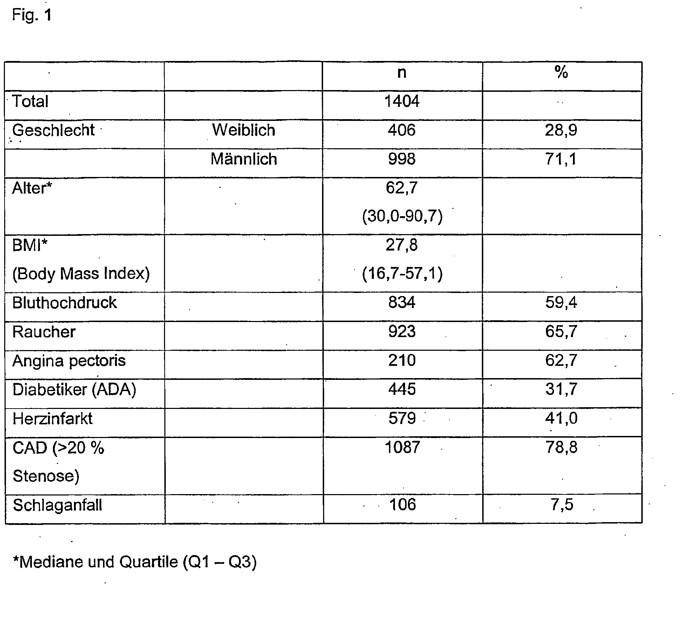

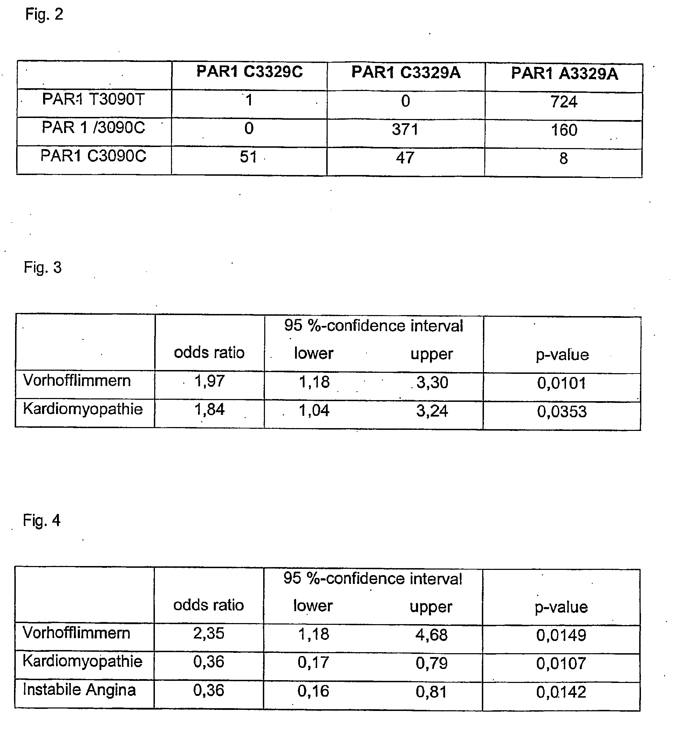

In der analysierten Patientengruppe (Fig. 1) zeigten sich statistisch signifikante Assoziationen der homozygoten Träger der PAR1 Variante C3090C mit Vorhofflimmern und Kardiomyopathie. Nach der Durchführung einer logistischen Regression konnte ein 1,97-fach erhöhtes Risiko für Vorhofflimmern und ein 1,84-fach erhöhtes Risiko für Kardiomyopathie bei homozygoten Trägern der PAR1 Variante C3090C gegenüber Trägern der PAR1 Varianten T3090/T3090T festgestellt werden (Fig. 3).
Für Träger der PAR1 Variante C3329C konnte gezeigt werden, dass diese Variante mit einem 2,35-fach erhöhten Risiko für Vorhofflimmern gegenüber Trägern der PAR1 Varianten C3329A/A3329A assoziiert. Bei Trägern der PAR1 Variante C3329C scheint diese Variante darüber hinaus protektiv für das Auftreten von akutem Koronarsyndrom und instabiler Angina zu sein. Gegenüber Trägern der PAR1 Varianten A3329C/A3329A haben Träger der PAR1 Variante C3329C ein 2,78-fach erniedrigtes Risiko für das Auftreten von akutem Koronarsyndrom und/oder instabiler Angina (Fig. 4).

Mittels eines erfindungsgemäßen Verfahrens unter Verwendung einer isolierten PAR1 Sequenz des jeweiligen SNP-Typs oder eines Fragments davon kann deshalb für menschliche Individuen ermittelt werden, ob eine Zuordnung zu einer Risikogruppe im Sinne der vorgestellten Resultate besteht.

Präparation von Plasmid-DNA

Es werden 1 ml einer Bakterien-Übernachtkultur in ein Eppendorf-Gefäß überführt und in einer Heraeus-Biofuge abzentrifugiert (5 min bei 5000rpm). Das Bakterienzellsediment ist in 100 µl gekühlter Lösung I zu resuspendieren und anschließend 5 min auf Eis zu stellen.

Lösung I: 25 mM Tris-HCl, PH 8,0, 50 mM Glucose (steril filtriert), 10 mM EDTA 100 µg/ml Rnase A,

Nach der Zugabe von 200 µl Lösung II wird gut gemischt. Es kommt hierbei zur alkalischen Denaturierung der DNA.

Lösung II: 200 mM NaOH, 1 % SDS.

Nach anschließender Inkubation für 5 min auf Eis werden dem Ansatz 150 µl Lösung III hinzugefügt. Es wird erneut gemischt und für weitere 15 min auf Eis inkubiert.

Lösung III: 3 M Natriumacetat (pH 4,8).

Durch eine 15-minütige Zentrifugation in der Biofuge Heraeus bei 12000 rpm werden die Zelltrümmer, die genomische DNA und die denaturierten Proteine abzentrifugiert. Der entstandene Überstand, in dem sich die Plasmid-DNA befindet, wird in ein zweites Eppendorf-Gefäß dekantiert und mit 1 ml 96 %-igem EtOH (bzw. 300 µl Isopropanol) versetzt. Der Fällungsansatz wird gründlich gemischt und erneut zentrifugiert (15 min bei 12000 rpm in der Biofuge Heraeus). Hierbei tritt die Fällung der Plasmid-DNA ein. Die sedimentierte Plasmid-DNA wird mit eiskaltem 70 %igem EtOH gewaschen und anschließend an der Luft getrocknet. Abschließend wird das trockene Sediment in 50 µl sterilem dest. Wasser aufgenommen.

Alkoholfällung von DNA

Fällungsansatz: DNA-Lösung, 1/10 Volumen 3 M Natriumacetat (pH 5,4), 2 bis 3 Volumina EtOH 96 % (1 Volumen Isopropanol).

Der Ansatz wird gut gemischt. Dieser kann bei -20°C aufbewahrt werden, dadurch wird die Fällungsausbeute jedoch nicht erhöht. Durch eine 20-minütige Zentrifugation bei 12000 rpm wird die Plasmid-DNA sedimentiert.

Um die Reste des eingesetzten Natriumacetats zu beseitigen, ist nach der Fällung die Plasmid-DNA noch einmal mit 1 ml 70 %igem EtOH zu waschen.

Phenolextraktion von DNA

Eine DNA-Lösung mit dem gleichen Volumen Phenol (Rotiphenol®, äquilibriert gegen TE-Puffer, pH 7,6; Roth, Karlsruhe) versetzt, für 5 min geschüttelt und bei 5000 rpm zentrifugiert. Der größte Teil der jetzt denaturierten Proteine sammelt sich in der Interphase. Die obere, wässrige Phase enthält die DNA und wird sorgfältig abgezogen, anschließend mit einem Chlorophorm/Isoamylalkoholgemisch (24:1) vermischt, um die Phenolreste zu entfernen. Danach wird erneut zentrifugiert, der wässrige Überstand abgenommen und durch Alkoholfällung die DNA aus der Lösung isoliert.

Aufreinigung von amplifizierten DNA-Molekülen

Die Aufreinigung von DNA-Amplifikaten erfolgt mit dem PCR-Purification-Kit (Qiagen). Dabei werden die Startermoleküle, Nukleotide (dNTPs), Polymerasen und Salze entfernt. Hierfür wird der PCR-Reaktionsansatz mit Fünffacher Volumen PB-Puffer versetzt, gut gemischt und auf die Qiaquick-Säule gegeben. Es folgt die selektive Bindung der amplifizierten DNA an das Säulenmaterial, die dNTPs werden durch zweimaliges Waschen mit 750 µl PE-Puffer entfernt. Anschließend wird die amplifizierte DNA mit dem gewünschten Wasservolumen eluiert, am besten mit dem gleichen Volumen wie beim Ausgangsmaterial des PCR-Reaktionsansatzes.

Spaltung von DNA mit Restriktionsenzymen

Ansatz: 3 µl DNA, 2 µl 10 x Spaltungspuffer, 2;5-5 U Restriktionsenzym (z.B. EcoRI, BamHI, SalI, XbaI, XhoI und andere), auf 20 µl Volumen mit dest. Wasser auffüllen.

Die Spaltungsreaktion läuft je nach Restriktionsenzym bei 25-55°C für 1-2 h ab. Zur Analyse werden die Fragmente in einem Agarose- oder Polyacrylamidgel parallel zu einem Längenstandard elektrophoretisch aufgetrennt. Handelt es sich um eine Doppelspaltung, so wird dem Ansatz zuerst ein Enzym zugegeben. Nach einer Stunde trägt man ein Aliquot auf ein entsprechendes Gel auf, ist die Spaltung erfolgt, so kann das zweite Enzym zugegeben werden. Spaltet das zweite Enzym nicht im gleichen Spaltungspuffer, so ist zuerst eine Alkoholfällung erforderlich.

Agarose-Gelelektrophorese von DNA

Die Agarose (Roth) wird in gewünschter Konzentration in 1 x Agarosepuffer gelöst und in einem Mikrowellengerät bis zum vollständigen Lösen der Agarose aufgekocht. Die Lösung wird anschließend in eine abgedichtete Flachgelkammer aus Plexiglas gegossen.
Die DNA-Proben werden mit 1/10 Volumen Ladeblau vermischt (50 % v/v Glycerin; 50 mM EDTA; 0,005 % w/v BPB [Merck, Darmstadt] und 0,005 % Xylencyanol) und in die Geltaschen pipettiert, die durch einen Kamm erzeugt werden. Die Elektrophorese erfolgt horizontal bei einer konstanten Spannung von 80-140 V, je nach Gelgröße bzw. Elektrodenabstand, in 1 x Agarosepuffer als Laufpuffer.

1 x Agarosepuffer: 40 mM Tris-HCl (pH 7,8), 5 mM Natriumacetat, 1mM EDTA.

Polyacrylamid-Gelelektrophorese von DNA

7,5 % Polyacrylamidgel-Lösung: 0,94 ml 40 %iger Acrylamid-Bisacrylamid-Stammlösung, 0,5 ml 10 x TBE-Puffer (400 mM Tris-HCl, pH 8,3; 200 mM Na-acetat, 20 mM EDTA), 0,25 ml 1 % AMPS, 10 µl TEMED, 3,33 ml dest. Wasser;

Diese Mischung wird zur Polymerisation (ca. 10-20 min) zwischen gut gereinigte, vertikale Glasplatten gegeben, die in Vertikalapparaturen eingespannt sind. Das Gel wird in 1 x TBE-Puffer bei einer konstanten Spannung von 140 V gefahren.

Sequenzierung von DNA

1-2 µg DNA sind in 81 µl dest. $H_2O$ zu lösen und 9 µl NaOH (2 N) zur Denaturierung zuzugeben. Nach 10-minütiger Inkubation bei Raumtemperatur wird der Ansatz präzipitiert, wobei ein gründliches Waschen des erhaltenen DNA-Sediments mit eiskaltem 80 %igem Ethanol wichtig für die nachfolgenden Sequenzreaktionen ist. Das Sediment ist mit 2 µl 5 x Sequenase-Puffer (200 mM Tris-Cl pH 7,5/100 mM $MgCl_2$/250 mM NaCl), 1 µl Oligonukleotid (1 pM/µl) und schließlich mit dest. $H_2O$ auf ein Gesamtvolumen von 10 µl aufzufüllen. Während der anschließenden 30-minütigen Inkubation im 37°C-Wasserbad kommt es zum Hybridisieren des Starter-Oligonukleotids an die DNA.

Für die Sequenzzierungsreaktion dem Hybridisierungsansatz zugesetzte Reagenzien: 1,0 µl DTT (0,1 M), 2,0 µl Markierungsgemisch (1:5 verdünnt), 0,5 µl [$\alpha$-$^{35}$S]dATP, 2 µl Sequenase™ (13 U/µl, United States Biochemical), (1: 8 mit Enzymverdünnungspuffer verdünnt).

Während der nachfolgenden 5-minütigen Inkubation bei Raumtemperatur findet die Synthese des Gegenstranges statt, wobei durch den Einbau des radioaktiv markierten dATPs, die Markierung der synthetischen DNA erfolgt. Anschließend werden jeweils 3,5 µl des Markierungsansatzes zu 2,5 µl der vier verschiedenen Terminnationsgemische gegeben. Eine erneute 5-minütige Inkubation bei 37°C führt zu den statistisch verteilten Abbruchreaktionen der Gegenstrangsynthese. Die Reaktionen werden durch Zugabe von 4 µl Stop-Puffer beendet, die Ansätze werden bei 80-90°C denaturiert und anschließend auf ein 6 %iges, denaturiertes Sequenzgel aufgetragen. Nach dem Laden der Proben findet der Hauptlauf für 2-5 h bei 30-50 W bzw. 1300-1600 V statt. Das Gel wird danach in einem 10 %igen Essigsäurebad fixiert (15 min), unter fließendem Wasser von Harnstoffresten befreit und anschließend getrocknet (45 min mit einem Warmluftfön oder 2 h im 70°C-Brutschrank). Die anschließende Autoradiographie erfolgt für 16-24 h bei 4°C (Fujii Medical X-ray-Film RX, 30 x 40; Kodak Scientific Imaging Film X-omat AR).

Markierungsgemisch-Stammlösung: Je 7,5 µM dATP, dTTP, dGTP, dCTP

Terminationsgemische: Je 80 µM dATP, dTTP, dGTP, dCTP und je 8 µM des jeweiligen ddNTP

Sequenase-Verdünnungspuffer: 10 mM Tris/HCl; pH 7,5, 5 mM DTT, 0,5 mg/ml BSA

Stop-Puffer: 95 % Formamid, 20 mM EDTA, 0,005 % (w/v) Xylencyanol FF

Automatische DNA-Sequenzierung

Ansatz: 1 µg Plasmid-DNA (bei PCR-Fragmenten gilt z.B. 100 ng/500 Nukleotide), 3-5 pmol Startermolekül (PCR-Primer, möglichst eine Tm von 55°C), 4 µl Dye Terminator ready-mix (FddNTPs-Ampli-TaqFS-Gemisch), auf 20 µl mit dest. Wasser auffüllen

Die PCR-Reaktion [25 x (15 sec. bei 94°C, 15 sec. bei 50°C, 4 min bei 60°C] wird mit Alkohol gefällt und in 4 µl Ladepuffer aufgenommen. Nun werden die Proben für 3 min. bei 95°C denaturiert, abzentrifugiert, und auf ein vertikales Polyacrylamidgel (34 cm lang, mit 24 parallelen Spuren versehen) aufgetragen.

Nach Anregung durch einen Argon-Laserstrahl bei 488 nm emittieren die Farbstoffe Licht verschiedener Wellenlängen zwischen 525 nm und 605 nm, das über ein Gitter, einen sog. Spektrographen, in seine Spektralfarben zerlegt wird. Anschließend erfolgt die zeitgleiche Detektion der Spektralfarben mit Hilfe des hochauflösenden Pixelfeldes einer CCD-Kamera. Die Erfassung der Daten erfolgt mit Hilfe eines Computers (Macintosh Quadra/650 MacIlcx Apple Share) und der entsprechenden Auswerte-Software (PE Biosystems, Weiterstadt).

Sequenzgel: 30 g Harnstoff (Sigma), 21,5 ml dest. $H_2O$, 6 ml 10 x TBE Das Gemisch wird in einem Weithalskolben bei 50°C auf dem Heizblock gelöst und folgendes zugegeben: 9 ml 40 % Bisacrylamid (filtriert), 180 µl 10 % APS, 24 µl Temed

Polymerase-Ketten-Reaktion (PCR Reaktion)

Folgende DNA-Polymerasen können verwendet werden:

Taq (Thermus aquaticus) DNA-Polymerase (rekombinant, Gibco/BRL) und 10 x PCR-Puffer

[200 mM Tris/HCl (pH 8,4), 500 mM KCl]

Tfl (Thermus flavus) DNA-Polymerase (Master Amp™, Biozym, Oldendorf) und 20 x PCR

Puffer [20 mM $(NH_2)SO_4$, 1 M Tris/HCl (pH 9,0)

PCR Reaktionsansatz:

| **PCR-Komponenten** | **Menge** |
|---|---|
| DNA-Matrize | 10-100ng |
| Startermolekül 1 | 25 μM |
| Startermolekül 2 | 25 μM |
| Nukleotidgemisch (dNTPs) | 20 mM (aus einem Gemisch mit je 10 mM d'NTP) |
| DNA-Polymerase Puffer | 1x:5,0 μl bei Taq DNA-Polymerase Puffer<br>2,5μl bei Tfl DNA-Polymerase Puffer |
| $MgCl_2$ | 75 mM |
| DNA-Polymerase | 2 U bei Taq DNA-Polymerase |
| | 1 U bei Tfl DNA-Polymerase |
| dest. $H_2O$ | auf ein Gesamtvolumen von 50 μl auffüllen |

Hierbei gilt: 1 U katalysiert den Umbau von 10 nM Desoxyribonukleosid-Triphosphaten bei 74°C innerhalb von 30 min in ein säureunlösliches DNA-Produkt.
Die PCR-Reaktion fängt normalerweise mit dem sog. Hot-start an: Erst wird der Ansatz ohne die Polymerase bei 94°C inkubiert, um die erste Denaturierung der DNA zu ermöglichen. Nach Erreichen einer Temperatur von 80°C wird die DNA-Polymerase zum Ansatz zugegeben, um eine unspezifische Amplifikation bei noch niedriger Temperatur zu vermeiden. Danach läuft die eigentliche PCR-Reaktion über 25-35 Zyklen ab.
Für jeden Zyklus gelten folgende Reaktionsbedingungen:

| **Reaktion** | **Temperatur** | **Zeit** |
|---|---|---|
| Denaturierung | 94°C | 30-60 sec |
| Hybridisierung (anealing) | $T_m$-5°C | 30-60 sec |
| Verlängerung (extension) | 72°C | 1 min/1kb |

Schließlich wird noch bei 72°C über 10 min die Kettenverlängerung durchgeführt und schließlich abgekühlt.

Isolierung von Gesamt-RNA

Alle Zentrifugationsschritte laufen bei 13000 rpm und 16°C ab.
Zellen werden mit 600μl Lysispuffer (100 RLT-Puffer: 1 Mercaptoethanol) lysiert. Das Zellysat wird auf eine QiaSchredder-Säule gegeben und für 2 min abzentrifugiert. Das Eluat wird mit 600 μl 70 % Ethanol versetzt, gut gemischt, DNA auf eine RNAeasy mini Spin-Säule gegeben und für 15 sec zentrifugiert (es erfolgt die RNA-Bindung an die Silica-Matrix). Die Säule wird dreimal gewaschen (einmal mit 700 ml RW1 Puffer und zweimal mit 500 μl RPE Puffer). Anschließend wird die Säule in ein autoklaviertes 1,5 ml Eppendorf-Gefäß überführt und die RNA wird mit 15 μl dest. $H_2O$ eluiert. Die

durchschnittliche Konzentration der auf diese Weise gewonnenen Gesamt-RNA beträgt 1µg/µl.

Auftrennung von RNA durch Agarose-Gelelektrophorese

Denaturierendes Agarose-Gel:

1 g Agarose, 37 ml dest. Wasser, 10 ml 10x MOPS (0,2 mM MOPS, 10 mM EDTA, 100 mM NaAc),
Das Gemisch wird aufgekocht und auf 60°C abgekühlt
16 ml 37 %iges Formaldehyd wird zugegeben.

Nach dem Erstarren wird es mit RNA-Gel-Laufpuffer in die Elektrophorese-Apparatur eingesetzt. Die RNA wird mit einem speziellen Probenpuffer zusammen aufgetragen.

RNA-Gel-Laufpuffer: 40 ml 10x MOPS, 65 ml 37 %iges Formaldehyd, 295 ml dest. Wasser

RNA-Probenpuffer: 1-5 µg RNA, 5 µl RNA-NEW-Puffer (7,5 µl 37 %iges Formaldehyd, 4,5 µl 10x MOPS, 25,9 µl Formamid, 7,5 µl dest. Wasser), 2 µl Formamid-Farbmarker [50 % (v/v) Glycerol, 1 mM EDTA (pH 8,0), 0,25 % (v/v) Bromphenolblau, 0,25 % (v/v) Xylencyanol].

Das Gel läuft ca. 3 h bei 80 V. Da es sich in dieser Arbeit ausschließlich um Eukaryonten RNA-Isolate handelt, müssen auf dem Gel die 28S- und 18S-rRNA als dominante Banden sichtbar sein.

Reverse Transkriptase mit MMLV-RT
(Moloney murine leukemia virus - Reverse Transkriptase)
Reverse Transkriptase-Ansatz: 5 µg RNA, 100 µM Startermolekül

Die RNA-Präparation und das Startermolekül werden für 10 min bei 75°C inkubiert, um mögliche Sekundärstrukturbildungen in der RNA-Matrize als Störfaktor für die Transkriptase zu vermeiden. Aber auch ohne diesen Schritt erfolgt normalerweise eine Transkriptionsreaktion.

Reverse Transkriptase-Reaktionsansatz: 28 U Rnasin® (Promega), 25 mM dNTPs, 5 µl 10x Reverse Transkriptase-Puffer [10 mM Tris/HCl (pH 8,3), 75 mM KCl, 3 mM $MgCl_2$], 50 U Reverse Transkriptase(StrataScript™, Stratagene), auf 50 µl Volumen mit dest. Wasser auffüllen.

Für die reverse Transkription wird der Ansatz 15 min bei 42°C und 45 min bei 37°C inkubiert. Eine längere Inkubation bei 42°C für 2 h mit einer 30-sekundigen Unterbrechung bei 55°C ist bei längeren RNA-Matrizen empfehlenswert. Eine anschließende 5-minütige Inkubation des Ansatzes bei 95°C führt zur Inaktivierung der Reversen Transkriptase. Für eine PCR-Reaktion werden dann 5-20 µl des Reversen Transkriptionsansatzes eingesetzt.

Präparation genomischer DNA aus Gewebe

Es werden 100 mg Gewebe in flüssigem Stickstoff zu Pulver zerstoßen. Das Gewebepuder wird in ein Falcontube mit 6 ml Reaktionspuffer (frisch 30 µl Proteinase K [20 mg/ml] dem Puffer zugegeben) gefüllt und über Nacht (12-18 Stunden) bei 56°C vorsichtig schüttelnd inkubiert. Nach der Inkubation werden 100 µl RNase A (10 µg/µl) zugegeben und eine Stunde bei 37°C unter weiterem Schütteln inkubiert.
Im Anschluß werden 4 ml Phenol zugefügt und das Tube wird etwa 5 min lang von Hand kopfüber gekippt. Umgehend werden 4 ml CI (Chloroform/Isoamylalkohol) zugegeben und das 5-minütige Kippen wiederholt und dann 15 min zentrifugiert (3000 rpm). Der Überstand wird vorsichtig abgenommen und in 10 ml Falcontubes überführt. Ist der Überstand noch nicht klar, muß die Phenolextraktion wiederholt werden, ansonsten werden erneut 4 ml CI zugegeben und das Tube 5 min lang von Hand über Kopf gekippt und danach 15 min zentrifugiert (3000 rpm). Der Überstand wird vorsichtig abgenommen und die CI-Extraktion wiederholt. Zu dem zum Schluß gewonnenen Überstand werden 1/10 Volumen Natriumazetatlösung (3 M, pH 6) und 2,5-faches Volumen Ethanol (99,8 %) zugegeben. Das Tube wird vorsichtig gedreht bis die DNA als Knäuel ausfällt. Dieses DNA-Knäuel wird mit Hilfe eines Glashakens in etwa 25 ml Ethanol (70 %) überführt und 3 min ruhen gelassen. Das Waschen wurde zweimal wiederholt. Danach wurde die DNA luftgetrocknet und bei Raumtemperatur in 0,5 ml Aqua bidestillata gelöst.

Southern Blot

Auftrennung der DNA durch ein Agarosegel

Gel zum Strangbruch der größeren DNA-Moleküle (> 6kBp) etwa 5 min auf kurzwelligem UV liegen lassen.
Gel zur DNA-Denaturierung für 30 min in Denaturierungslösung schwenken.
Gel zur Neutralisierung für 30 min in Neutralisierungslösung schwenken.

Blotaufbau (von unten nach oben): Gel, Nylonmembran, trockenes Filterpapier, saugfähiges Papier, Platte, Gewicht (ca. 1 kg).
Blotting über Nacht mit 20 x SSC.

Membran für 10 min in 2 x SSC waschen
Membran auf Filterpapier trocknen
Fixieren der Nukleinsäure durch Backen bei 80°C für 1 h oder UV-Crosslinking (e.g. im "Stratalinker", Automatic position).
Membran kann dann bis zur Hybridisierung aufbewahrt werden.
Prähybridisierung der Membran in der Hybridisierungslösung für ca. 1 - 2 h Abdecken unspezifischer Bindungsstellen auf der Membran.

Hybridisierungslösung: 5 x SSC, 5 x Denhardt's Lösung, 0,5 % SDS, 100 μg/ml Heringssperma-DNA,

Denaturierungslösung: 0,5 M NaOH (20 g), 1 M NaCl

Neutralisierungslösung: 1,5 M NaCl/0,5 M Tris pH 7,4

20 x SSC ist 3 M NaCl, 0,3 M Na-Citrat: 175,3 g NaCl, 88,2 g Na-Citrat X 2 $H_2O$ ad 1 l bidest. Wasser, pH auf 7,0 mit HCl einstellen

50 x Denhardt's Lösung: 5 g Ficoll 400, 5 g PVP (Polyvinylpyrrolidon), 5 g BSA, bidest. Wasser ad 500 ml

Northern Blot

Auftrennung der RNA mittels Formaldehyd-Agarosegel

Blotaufbau (von unten nach oben): Gel, Nylonmembran, trockenes Filterpapier, saugfähiges Papier, Platte, Gewicht (ca. 1 kg).
Blotting über Nacht mit 20 x SSC.
RNA auf Filter durch Backen bei 80°C (1 h) fixieren
Filter zur Deglyoxylierung der RNA in kochendes 20 mM Tris pH 8 geben und auf RT abkühlen lassen.

Beschreibung der Figuren

Fig. 1
Charakteristika der Studiengruppe

Fig. 2
Verteilung der PAR1 Varianten T3090C und A3329C in 1362 analysierten Individuen

Fig. 3
Assoziation der PAR1 Varianten C3090C mit Vorhofflimmern und Kardiomyopathie

Fig. 4
Assoziation der PAR1 Varianten C3329C mit Vorhofflimmern, akutem Koronarsyndrom und instabiler Angina.

Fig. 5
Polynukleotidsequenz der cDNA des humanen PAR 1 Gens in 5'/3'-Anordnung. Die Sequenz entspricht der unter der Nummer NM_001992 von der NCBI Nucleotide Database der Öffentlichkeit zur Verfügung gestellten Sequenz. Die hergestellte Sequenz ist gleichlautend wie die SEQ ID Nr. 1.

Fig. 6
Polynukleotidsequenz der cDNA des humanen PAR1 Gens in 5'/3'-Anordnung mit einem Polymorphismus an Position 3090 der Sequenz gemäß NM_001992 bestehend aus einem Austausch von T nach C. Die dargestellte Sequenz ist gleichlautend wie die SEQ ID Nr. 2.

Fig. 7
Polynukleotidsequenz der cDNA des humanen PAR1 Gens in 5'/3'-Anordnung mit einem Polymorphismus an Position 3329 der Sequenz gemäß NM_001992 bestehend aus einem Austausch von A nach C. Die dargestellte Sequenz ist gleichlautend wie die SEQ ID Nr. 3.

Fig. 8
Polynukleotidsequenz der cDNA des humanen PAR1 Gens in 5'/3'-Anordnung mit einem Polymorphismus an Position 3090 der Sequenz gemäß NM_001992 bestehend aus einem Austausch von T nach C sowie einem gleichzeitig vorliegenden zweiten Polymorphismus an Position 3329 der Sequenz gemäß NM_001992 bestehend aus einem Austausch von A nach C. Die dargestellte Sequenz ist gleichlautend wie die SEQ ID Nr. 4.

Fig. 9
Polynukleotidsequenz eines Fragments des humanen PAR1 Gens in 5'/3'-Anordnung. Die dargestellte Sequenz ist gleichlautend wie die SEQ ID Nr. 5.

Fig. 10
Polynukleotidsequenz eines Fragments des humanen PAR1-Gens in 5'/3'-Anordnung mit einem Polymorphismus an Position 3090 der Sequenz gemäß NM_01992 bestehend aus einem Austausch von T nach C. Die dargestellte Sequenz ist gleichlautend wie die SEQ ID Nr. 6.

Fig. 11
Polynukleotidsequenz eines Fragmentsdes humanen PAR1-Gens in 5'/3'-Anordnung mit einem Polymorphismus an Position 3329 der Sequenz gemäß NM_001992 bestehend aus einem Austausch von A nach C. Die dargestellte Sequenz ist gleichlautend wie die SEQ ID Nr. 7.

Fig. 12
Polynukleotidsequenz eines Fragments des humanen PAR1-Gens in 5'/3'-Anordnung mit einem Polymorphismus an Position 3090 der Sequenz gemäß NM_001992 bestehend aus einem Austausch von T nach C sowie einem gleichzeitig vorliegenden zweiten Polymorphismus an Position 3329 der Sequenz gemäß NM_001992 bestehend aus einem Austausch von A nach C. Die dargestellte Sequenz ist gleichlautend wie die SEQ ID Nr. 8.

Fig. 13
Polynukleotidsequenz in 5'/3'-Anordnung vom 5'-Ende der cDNA des humanen PAR1-Gens. Die dargestellte Sequenz ist gleichlautend wie die SEQ ID Nr. 9.

Fig. 14
Polynukleotidsequenz in 5'/3'-Anordnung vom 3'-Ende der cDNA des humanen PAR1-Gens. Die dargestellte Sequenz ist gleichlautend wie die SEQ ID Nr. 10.

Fig. 15
Polynukleotidsequenz in 5'/3'-Anordnung der cDNA des humanen PAR1-Gens betreffend die Positionen 2767 bis 2789 gemäß NM_001992. Die dargestellte Sequenz ist gleichlautend wie die SEQ ID Nr. 11.

Fig. 16
Polynukleotidsequenz in 5'/3'-Anordnung aus dem Exon Nr. 1 des humanen PAR1-Gens. Die dargestellte Sequenz ist gleichlautend wie die SEQ ID Nr. 12.

Fig. 17
Proteinsequenz des humanen PAR1-Rezeptors. Die Sequenz entspricht der unter der Nummer NP_001983 von der NCBI Protein-Database der Öffentlichkeit zur Verfügung gestellten Sequenz. Die dargestellte Sequenz ist gleichlautend wie die SEQ ID. Nr. 13.

## Patentansprüche

1. Isoliert vorliegende Polynukleotidsequenz des PAR1 Gens, **dadurch gekennzeichnet, dass** an Position 3090 der Sequenz gemäß NM-001992 ein Austausch des T gegen ein C vorliegt.

2. Isoliert vorliegende Polynukleotidsequenz nach Anspruch 1, **dadurch gekennzeichnet, dass** die Polynukleotidsequenz des PAR1 Gens eine Sequenz gemäß SEQ ID NO: 2 umfasst.

3. Isoliert vorliegende Polynukleotidsequenz nach Anspruch 1, **dadurch gekennzeichnet, dass** die Polynukleotidsequenz des PAR1 Gens aus einer Sequenz gemäß SEQ ID NO: 2 besteht.

4. Isoliert vorliegende Polynukleotidsequenz des PAR1 Gens, **dadurch gekennzeichnet, dass** an Position 3329 der Sequenz gemäß NM_001992 ein Austausch des A gegen ein C vorliegt.

5. Isoliert vorliegende Polynukleotidsequenz nach Anspruch 4, **dadurch gekennzeichnet, dass** die Polynukleotidsequenz des PAR 1 Gens eine Sequenz gemäß SEQ ID Nr. 3 umfasst.

6. Isoliert vorliegende Polynukleotidsequenz nach Anspruch 4, **dadurch gekennzeichnet, dass** die Polynukleotidsequenz des PAR 1 Gens aus einer Sequenz gemäß SEQ ID Nr. 3 besteht.

7. Isoliert vorliegende Polynukleotidsequenz des PAR 1 Gens, **dadurch gekennzeichnet, dass** jeweils bezogen auf NM_001992 an Position 3090 ein Austausch des T gegen ein C und an Position 3329 ein Austausch des A gegen ein C vorliegt.

**8.** Isoliert vorliegende Polynukleotidsequenz nach Anspruch 7, **dadurch gekennzeichnet, dass** die Polynukleotidsequenz des PAR 1 Gens eine Sequenz gemäß SEQ ID Nr. 4 umfasst.

**9.** Isoliert vorliegende Polynukleotidsequenz nach Anspruch 7, **dadurch gekennzeichnet, dass** die Polynukleotidsequenz des PAR 1 Gens aus einer Sequenz gemäß SEQ ID Nr. 4 besteht.

**10.** Isoliert vorliegender Teil der Polynukleotidsequenz des PAR 1 Gens bestehend aus einer Sequenz gemäß SEQ ID Nr. 5.

**11.** Isoliert vorliegender Teil der Polynukleotidsequenz des PAR 1 Gens bestehend aus einer Sequenz gemäß SEQ ID Nr. 6.

**12.** Isoliert vorliegender Teil der Polynukleotidsequenz des PAR 1 Gens bestehend aus einer Sequenz gemäß SEQ ID Nr. 7.

**13.** Isoliert vorliegender Teil der Polynukleotidsequenz des PAR 1 Gens bestehend aus einer Sequenz gemäß SEQ ID Nr. 8.

**14.** Herstellung einer Polynukleotidsequenz gemäß einem der Ansprüche 1 bis 9 mittels der folgenden Verfahrensschritte:

a] Bereitstellung von humaner cDNA enthaltend eine PAR 1 Sequenz gemäß SEQ ID Nr. 2 und/oder eine PAR 1 Sequenz gemäß SEQ ID Nr. 3 und/oder eine PAR 1 Sequenz gemäß SEQ ID Nr. 4,
b] Bereitstellung eine Primerpaares gemäß SEQ ID Nr. 9 und SEQ ID Nr. 10.
c] Amplifizierung der PAR 1 Polynukleotidsequenz durch die Polymerasekettenverlängerungsreaktion (PCR),
d] Isolierung und/oder Reinigung des entstandenen Fragments mit einer Länge von 3,56 kb aus c],
e] Sequenzierung des Fragments aus d].

**15.** Herstellung einer Polynukleotidsequenz gemäß einem der Ansprüche 10 bis 13 mittels der folgenden Verfahrensschritte:

a] Bereitstellung von
humaner genomischer DNA enthaltend eine PAR 1 Sequenz gemäß SEQ ID Nr. 1 und/oder eine PAR 1 Sequenz gemäß SEQ ID Nr. 2 und/oder eine PAR 1 Sequenz gemäß SEQ ID Nr. 3 und/oder eine PAR 1 Sequenz gemäß SEQ ID Nr. 4
b] Bereitstellung eines Primerpaares gemäß SEQ ID Nr. 11 und SEQ ID Nr. 12
c] Amplifizierung des Fragments der PAR 1 Polynukleotidsequenz durch die Polymerasekettenverlängerungsreaktion (PCR),
d] Isolierung und/oder Reinigung des entstandenen Fragments aus c],
e] Sequenzierung des Fragments aus d].

**16.** Verfahren zum Nachweis, ob in einem PAR 1 Gen an Position 3090 der Sequenz gemäß NM_001992 ein Austausch von T nach C und/oder an Position 3329 der Sequenz gemäß NM_001992 ein Austausch von A nach C vorliegt oder nicht vorliegt, umfassend die folgenden Verfahrensschritte:

a] Bereitstellung von biologischem Material enthaltend Zellen eines Menschen,
b] Gewinnung von chromosomaler DNA aus dem Material von a],
c] Amplifizierung eines Polynukleotidfragments mittels der Primer gemäß SEQ ID Nr. 11 und SEQ ID Nr. 12 durch eine PCR-Reaktion
d] Sequenzierung des Polynukleotidfragments aus c].

**17.** Verfahren zum Nachweis, ob in einem PAR 1 Gen an Position 3090 der Sequenz gemäß NM_001992 ein Austausch von T nach C und/oder an Position 3329 der Sequenz gemäß NM_001992 ein Austausch von A nach C vorliegt oder nicht vorliegt, umfassend die folgenden Verfahrensschritte:

a] Bereitstellung von biologischem Material enthaltend Zellen eines Menschen,

b] Gewinnung von RNA aus dem Material von a],
c] Umschreiben der RNA in cDNA mittels reverser Transkriptase,
d] eventuell Amplifizierung eines Polynukleotidfragments mittels der Primer gemäß SEQ ID Nr. 10 und SEQ ID Nr. 11 durch die PCR-Reaktion ,
e] Sequenzzierung der cDNA aus c] und/oder des Polynukleotidfragments aus d].

**18.** Verfahren zum Nachweis, ob in einem PAR 1 Gen an Position 3090 der Sequenz gemäß NM_001992 ein Austausch von T nach C und/oder an Position 3329 der Sequenz gemäß NM_001992 ein Austausch von A nach C vorliegt oder nicht vorliegt, umfassend die folgenden Verfahrensschritte:

a] Bereitstellung von biologischem Material enthaltend Zellen eines Menschen,
b] Gewinnung von chromosomaler DNA aus dem Material von a],
c] Southern-Blotting der chromosonalen DNA aus b]
d] Bereitstellung einer Sonde gemäß SEQ ID Nr. 5 und/oder SEQ ID Nr. 6 und/oder SEQ ID Nr. 7 und/oder SEQ ID Nr. 8,
e] Hybridisierung des Southern Blots aus c] mit der Sonde aus d] unter stringenten Hybridisierungsbedingungen,
f] Bestimmung des Vorliegens oder Nichtvorliegens einer genetischen Variation im Gen PAR 1 an Position 3090 und/oder 3329 gemäß NM_001992 durch Vergleich der Ergebnisse der Hybridisierung aus e].

**19.** Verfahren zum Nachweis, ob in einem PAR 1 Gen an Position 3090 der Sequenz gemäß NM_001992 ein Austausch von T nach C und/oder an Position 3329 der Sequenz gemäß NM_001992 ein Austausch von A nach C vorliegt oder nicht vorliegt, umfassend die folgenden Verfahrensschritte

a] Bereitstellung von biologischem Material enthaltend Zellen eines Menschen,
b] Gewinnung von RNA aus dem Material von a]
c] Bereitstellung einer Sonde gemäß SEQ ID Nr. 5 und/oder SEQ ID Nr. 6 und/oder SEQ ID Nr. 7 und/oder SEQ ID Nr. 8,
e] Hybridisierung des Northern Blots aus c] mit der Sonde aus d] unter stringenten Hybridisierungsbedingugnen,
f] Bestimmung des Vorliegens oder Nichtvorliegens einer genetischen Variation im Gen PAR 1 an Position 3090 und/oder 3329 gemäß NM_001992 durch Vergleich der Ergebnisse der Hybridisierung.

**20.** Isolierte Polynukleotidsequenz mit einer Anzahl von 21 bis 50 Nukleotiden, **gekennzeichnet dadurch, dass** eine Sequenz gemäß SEQ ID Nr. 11 enthalten ist.

**21.** Isolierte Polynukleotidsequenz bestehend aus SEQ ID Nr. 11.

**22.** Isolierte Polynukleotidsequenz mit einer Anzahl von 21 bis 50 Nukleotiden, **gekennzeichnet dadurch, dass** eine Sequenz gemäß SEQ ID Nr. 12 enthalten ist.

**23.** Isolierte Polynukleotidsequenz bestehend aus SEQ ID Nr. 12.

**24.** Verwendung einer isolierten Polynukleotidsequenz gemäß Anspruch 20 oder 21 in Kombination mit einer isolierten Polynukleotidsequenz gemäß Anspruch 22 oder 23 zur Vermehrung eines Fragments aus dem PAR 1 Gen mittels der PCR-Reaktion.

**25.** Verwendung nach Anspruch 24, **gekennzeichnet dadurch, dass** das PAR 1 Gen an Position 3090 der Sequenz gemäß NM_001992 einen Austausch von T nach C aufweist und/oder an Position 3329 der Sequenz gemäß NM_001992 einen Austausch von A nach C aufweist.

**26.** Kit enthaltend

a] eine Polynukleotidsequenz gemäß Anspruch 20 oder 21,
b] eine Polynukleotidsequenz gemäß Anspruch 22 oder 23,
c] mindestens ein Enzym für die Durchführung der PCR-Reaktion
d] sowie eventuell Substanzen und/oder Lösungen für die Ausführung der Polymerasekettenverlängerungsreaktion (PCR),
e] sowie eventuell weiterhin Polynukleotidsequenzen gemäß einem oder mehreren der Ansprüche 1 bis 17
f] sowie eventuell Reagenzien zur Durchführung einer Sequenzzierung.

**27.** Herstellung des Kit gemäß Anspruch 26 wobei

a] eine Polynukleotidsequenz gemäß Anspruch 20 oder 21 hergestellt wird,
b] eine Polynukleotidsequenz gemäß Anspruch 22 oder 23 bereitgestellt wird,
c] ein Enzym für die Durchführung der PCR-Reaktion bereitgestellt wird,
d] gegebenenfalls Reagenzien zur Durchführung einer Sequenzzierung bereitgestellt werden
e] eventuell Substanzen und/oder Lösungen für die Ausführung der Polymerasekettenverlängerungsreaktion (PCR) bereitgestellt werden,
f] eventuell Polynukleotidsequenzen gemäß einem oder mehreren der Ansprüche 1 bis 13 bereitgestellt werden,
g] die Komponenten aus a] bis e] jeweils separat in geeignete Behältnisse gegeben werden,
h] die Behältnisse aus g] gegebenenfalls kombiniert in einer oder mehrerer Verpackungseinheiten zusammengefasst werden.

**28.** Verwendung des Kit gemäß Anspruch 26 oder 27 zur Vermehrung eines Fragments aus dem PAR 1 Gen sowie eventuell der weiteren Analyse, ob genetische Variationen im Gen PAR 1 an den Positionen 3090 und/oder 3329 gemäß NM_001992 vorliegen.

## Claims

**1.** An isolated polynucleotide sequence of the PAR1 gene, which comprises a C for T substitution at position 3090 of the sequence according to NM-001992.

**2.** The isolated polynucleotide sequence as claimed in claim 1, wherein the polynucleotide sequence of the PAR1 gene encompasses a sequence according to SEQ ID NO: 2.

**3.** The isolated polynucleotide sequence as claimed in claim 1, wherein the polynucleotide sequence of the PAR1 gene consists of a sequence according to SEQ ID NO: 2.

**4.** An isolated polynucleotide sequence of the PAR1 gene, which comprises a C for A substitution at position 3329 of the sequence according to NM-001992.

**5.** The isolated polynucleotide sequence as claimed in claim 4, wherein the polynucleotide sequence of the PAR1 gene encompasses a sequence according to SEQ ID NO: 3.

**6.** The isolated polynucleotide sequence as claimed in claim 4, wherein the polynucleotide sequence of the PAR1 gene consists of a sequence according to SEQ ID NO: 3.

**7.** An isolated polynucleotide sequence of the PAR1 gene, which comprises a C for T substitution at position 3090 and a C for A substitution at position 3329, in each case based on NM-001992.

**8.** The isolated polynucleotide sequence as claimed in claim 7, wherein the polynucleotide sequence of the PAR1 gene encompasses a sequence according to SEQ ID NO: 4.

**9.** The isolated polynucleotide sequence as claimed in claim 7, wherein the polynucleotide sequence of the PAR1 gene consists of a sequence according to SEQ ID NO: 4.

**10.** An isolated part of the polynucleotide sequence of the PAR1 gene, consisting of a sequence according to SEQ ID NO: 5.

**11.** An isolated part of the polynucleotide sequence of the PAR1 gene, consisting of a sequence according to SEQ ID NO: 6.

**12.** An isolated part of the polynucleotide sequence of the PAR1 gene, consisting of a sequence according to SEQ ID NO: 7.

**13.** An isolated part of the polynucleotide sequence of the PAR1 gene, consisting of a sequence according to SEQ ID NO: 8.

**14.** A method for preparing a polynucleotide sequence as claimed in any of claims 1 to 9 by means of the following method steps:

a] Providing human cDNA comprising a PAR1 sequence according to SEQ ID NO: 2 and/or a PAR1 sequence according to SEQ ID NO: 3 and/or a PAR1 sequence according to SEQ ID NO: 4,
b] Providing a primer pair according to SEQ ID NO: 9 and SEQ ID NO: 10.
c] Amplifying the PAR1 polynucleotide sequence by the polymerase chain extension reaction (PCR),
d] Isolating and/or purifying the 3.56 kb fragment obtained from c],
e] Sequencing the fragment from d].

**15.** A method for preparing a polynucleotide sequence as claimed in any of claims 10 to 13 by means of the following method steps:

a] Providing human genomic DNA comprising a PAR1 sequence according to SEQ ID NO: 1 and/or a PAR1 sequence according to SEQ ID NO: 2 and/or a PAR1 sequence according to SEQ ID NO: 3 and/or a PAR1 sequence according to SEQ ID NO: 4
b] Providing a primer pair according to SEQ ID NO: 11 and SEQ ID NO: 12
c] Amplifying the fragment of the PAR1 polynucleotide sequence by the polymerase chain extension reaction (PCR),
d] Isolating and/or purifying the fragment obtained from c],
e] Sequencing the fragment from d].

**16.** A method for detecting whether or not there is in a PAR1 gene a T to C substitution at position 3090 of the sequence according to NM-001992 and/or an A to C substitution at position 3329 of the sequence according to NM-001992, which method comprises the following method steps:

a] Providing biological material comprising human cells,
b] Obtaining chromosomal DNA from the material of a],
c] Amplifying a polynucleotide fragment by means of the primers according to SEQ ID NO: 11 and SEQ ID NO: 12, using a PCR reaction,
d] Sequencing the polynucleotide fragment from c] .

**17.** A method for detecting whether or not there is in a PAR1 gene a T to C substitution at position 3090 of the sequence according to NM-001992 and/or an A to C substitution at position 3329 of the sequence according to NM-001992, which method comprises the following method steps:

a] Providing biological material comprising human cells,
b] Obtaining RNA from the material of a],
c] Transcribing said RNA into cDNA by means of reverse transcriptase,
d] Possibly amplifying a polynucleotide fragment by means of the primers according to SEQ ID NO: 10 and SEQ ID NO: 11, using the PCR reaction,
e] Sequencing the cDNA from c] and/or the polynucleotide fragment from d].

**18.** A method for detecting whether or not there is in a PAR1 gene a T to C substitution at position 3090 of the sequence according to NM-001992 and/or an A to C substitution at position 3329 of the sequence according to NM-001992, which method comprises the following method steps:

a] Providing biological material comprising human cells,
b] Obtaining chromosomal DNA from the material of a],
c] Southern blotting the chromosomal DNA from b],
d] Providing a probe according to SEQ ID NO: 5 and/or SEQ ID NO: 6 and/or SEQ ID NO: 7 and/or SEQ ID NO: 8,
e] Hybridizing the Southern blot from c] with the probe from d] under stringent hybridization conditions,
f] Determining the presence or absence of a genetic variation in the PAR1 gene at position 3090 and/or 3329 according to NM-001992 by comparing the results of the hybridization from e].

**19.** A method for detecting whether or not there is in a PAR1 gene a T to C substitution at position 3090 of the sequence according to NM-001992 and/or an A to C substitution at position 3329 of the sequence according to NM-001992, which method comprises the following method steps:

a] Providing biological material comprising human cells,
b] Obtaining RNA from the material of a],
c] Providing a probe according to SEQ ID NO: 5 and/or SEQ ID NO: 6 and/or SEQ ID NO: 7 and/or SEQ ID NO: 8,
e] Hybridizing the Northern blot from c] with the probe from d] under stringent hybridization conditions,
f] Determining the presence or absence of a genetic variation in the PAR1 gene at position 3090 and/or 3329 according to NM-001992 by comparing the results of the hybridization.

**20.** An isolated polynucleotide sequence having from 21 to 50 nucleotides, which comprises a sequence according to SEQ ID NO: 11.

**21.** An isolated polynucleotide sequence consisting of SEQ ID NO: 11.

**22.** An isolated polynucleotide sequence having from 21 to 50 nucleotides, which comprises a sequence according to SEQ ID NO: 12.

**23.** An isolated polynucleotide sequence comprising SEQ ID NO: 12.

**24.** The use of an isolated polynucleotide sequence as claimed in claim 20 or 21 in combination with an isolated polynucleotide sequence as claimed in claim 22 or 23 for amplifying a fragment of the PAR1 gene by means of the PCR reaction.

**25.** The use as claimed in claim 24, wherein the PAR1 gene has a T to C substitution at position 3090 of the sequence according to NM-001992 and/or an A to C substitution at position 3329 of the sequence according to NM-001992.

**26.** A kit, comprising

a] a polynucleotide sequence as claimed in claim 20 or 21,
b] a polynucleotide sequence as claimed in claim 22 or 23,
c] at least one enzyme for carrying out the PCR reaction
d] and possibly substances and/or solutions for carrying out the polymerase chain extension reaction (PCR),
e] and possibly furthermore polynucleotide sequences as claimed in one or more of claims 1 to 17
f] and possibly reagents for carrying out a sequencing.

**27.** A method for preparing the kit as claimed in claim 26, which method comprises

a] preparing a polynucleotide sequence as claimed in claim 20 or 21,
b] providing a polynucleotide sequence as claimed in claim 22 or 23,
c] providing an enzyme for carrying out the PCR reaction,
d] providing, where appropriate, reagents for carrying out a sequencing
e] possibly providing substances and/or solutions for carrying out the polymerase chain extension reaction (PCR),
f] possibly providing polynucleotide sequences as claimed in one or more of claims 1 to 13,
g] introducing the components from a] to e] in each case separately into suitable containers,
h] combining, where appropriate, the containers from g] in one or more pack-units.

**28.** The use of the kit as claimed in claim 26 or 27 for amplifying a fragment of the PAR1 gene and possibly for the further analysis, as to whether genetic variations are present in the PAR1 gene at positions 3090 and/or 3329 according to NM-001992.

## Revendications

**1.** Séquence polynucléotidique du gène PAR 1 se trouvant sous forme isolée, **caractérisée en ce qu'**en position 3090 de la séquence selon NM_001992 se trouve un remplacement de la T par une C.

**2.** Séquence polynucléotidique se trouvant sous forme isolée selon la revendication 1, **caractérisée en ce que** la séquence polynucléotidique du gène PAR 1 comprend une séquence selon la SEQ ID NO : 2.

**3.** Séquence polynucléotidique se trouvant sous forme isolée selon la revendication 1, **caractérisée en ce que** la séquence polynucléotidique du gène PAR 1 est constituée par une séquence selon la SEQ ID NO : 2.

**4.** Séquence polynucléotidique du gène PAR 1 se trouvant sous forme isolée, **caractérisée en ce qu'**en position 3329 de la séquence selon NM_001992 se trouve un remplacement de l'A par une C.

**5.** Séquence polynucléotidique se trouvant sous forme isolée selon la revendication 4, **caractérisée en ce que** la séquence polynucléotidique du gène PAR 1 comprend une séquence selon la SEQ ID NO : 3.

**6.** Séquence polynucléotidique se trouvant sous forme isolée selon la revendication 4, **caractérisée en ce que** la séquence polynucléotidique du gène PAR 1 est constituée par une séquence selon la SEQ ID NO : 3.

**7.** Séquence polynucléotidique du gène PAR 1 se trouvant sous forme isolée, **caractérisée en ce que**, à chaque fois par rapport au NM_001992, en position 3090 se trouve un remplacement de la T par une C et en position 3329 un remplacement de l'A par une C.

**8.** Séquence polynucléotidique se trouvant sous forme isolée selon la revendication 7, **caractérisée en ce que** la séquence polynucléotidique du gène PAR 1 comprend une séquence selon la SEQ ID NO : 4.

**9.** Séquence polynucléotidique se trouvant sous forme isolée selon la revendication 7, **caractérisée en ce que** la séquence polynucléotidique du gène PAR 1 est constituée par une séquence selon la SEQ ID NO : 4.

**10.** Partie se trouvant sous forme isolée de la séquence polynucléotidique du gène PAR 1 constituée par une séquence selon la SEQ ID NO : 5.

**11.** Partie se trouvant sous forme isolée de la séquence polynucléotidique du gène PAR 1 constituée par une séquence selon la SEQ ID NO : 6.

**12.** Partie se trouvant sous forme isolée de la séquence polynucléotidique du gène PAR 1 constituée par une séquence selon la SEQ ID NO : 7.

**13.** Partie se trouvant sous forme isolée de la séquence polynucléotidique du gène PAR 1 constituée par une séquence selon la SEQ ID NO : 8.

**14.** Préparation d'une séquence polynucléotidique selon l'une quelconque des revendications 1 à 9 au moyen des étapes de procédé suivantes :

a] mise à disposition d'ADNc humain contenant une séquence PAR 1 selon la SEQ ID NO : 2 et/ou une séquence PAR 1 selon la SEQ ID NO : 3 et/ou une séquence PAR 1 selon la SEQ ID NO : 4,
b] mise à disposition d'une paire d'amorces selon les SEQ ID NO : 9 et SEQ ID NO : 10,
c] amplification de la séquence polynucléotidique PAR 1 par la réaction d'allongement de chaîne par polymérase (PCR),
d] isolement et/ou purification du fragment formé d'une longueur de 3,56 kb de c],
e] séquençage du fragment de d].

**15.** Préparation d'une séquence polynucléotidique selon l'une quelconque des revendications 10 à 13 au moyen des étapes de procédé suivantes :

a] mise à disposition d'ADN génomique humain contenant une séquence PAR 1 selon la SEQ ID NO : 1 et/ou une séquence PAR 1 selon la SEQ ID NO : 2 et/ou une séquence PAR 1 selon la SEQ ID NO : 3 et/ou une séquence PAR 1 selon la SEQ ID NO : 4.
b] mise à disposition d'une paire d'amorces selon les SEQ ID NO : 11 et SEQ ID NO : 12,
c] amplification du fragment de la séquence polynucléotidique PAR 1 par la réaction d'allongement de chaîne par polymérase (PCR),
d] isolement et/ou purification du fragment formé dans c],
e] séquençage du fragment de d].

**16.** Procédé pour détecter s'il existe ou non dans un gène PAR 1 en position 3090 de la séquence selon NM_001992 un remplacement de la T par C et/ou en position 3329 de la séquence selon NM_001992 un remplacement d'A par C, comprenant les étapes de procédé suivantes :

a] mise à disposition de matière biologique contenant des cellules d'un être humain,
b] extraction d'ADN chromosomique de la matière de a],
c] amplification d'un fragment polynucléotidique au moyen des amorces selon les SEQ ID NO : 11 et SEQ ID NO : 12 par une réaction PCR,
d] séquençage du fragment polynucléotidique de c].

**17.** Procédé pour détecter s'il existe ou non dans un gène PAR 1 en position 3090 de la séquence selon NM_001992 un remplacement de la T par C et/ou en position 3329 de la séquence selon NM_001992 un remplacement d'A par C, comprenant les étapes de procédé suivantes :

a] mise à disposition de matière biologique contenant des cellules d'un être humain,
b] extraction d'ARN de la matière de a],
c] transcription de l'ARN en ADNc au moyen d'une transcriptase inverse,
d] amplification éventuelle d'un fragment polynucléotidique au moyen des amorces selon les SEQ ID NO : 10 et SEQ ID NO : 11 par la réaction PCR,
e] séquençage de l'ADNc de c] et/ou du fragment polynucléotidique de d].

**18.** Procédé pour détecter s'il existe ou non dans un gène PAR 1 en position 3090 de la séquence selon NM_001992 un remplacement de la T par C et/ou en position 3329 de la séquence selon NM_001992 un remplacement d'A par C, comprenant les étapes de procédé suivantes :

a] mise à disposition de matière biologique contenant des cellules d'un être humain,
b] extraction d'ADN chromosomique de la matière de a],
c] Southern-Blot de l'ADN chromosomique de b]
d] mise à disposition d'une sonde selon les SEQ ID NO : 5 et/ou SEQ ID NO : 6 et/ou SEQ ID NO : 7 et/ou SEQ ID NO : 8,
e] hybridation du Southern Blot de c] avec la sonde de d] dans des conditions d'hybridation stringentes,
f] détermination de la présence ou non d'une variation génétique dans le gène PAR 1 en position 3090 et/ou 3329 selon NM_001992 par comparaison des résultats de l'hybridation de e].

**19.** Procédé pour détecter s'il existe ou non dans un gène PAR 1 en position 3090 de la séquence selon NM_001992 un remplacement de la T par C et/ou en position 3329 de la séquence selon NM_001992 un remplacement d'A par C, comprenant les étapes de procédé suivantes :

a] mise à disposition de matière biologique contenant des cellules d'un être humain,
b] extraction d'ARN de la matière de a],
c] mise à disposition d'une sonde selon les SEQ ID NO : 5 et/ou SEQ ID NO : 6 et/ou SEQ ID NO : 7 et/ou SEQ ID NO : 8,
e] hybridation du Northern Blot de c] avec la sonde de d] dans des conditions d'hybridation stringentes,
f] détermination de la présence ou non d'une variation génétique dans le gène PAR 1 en position 3090 et/ou 3329 selon NM_001992 par comparaison des résultats de l'hybridation.

**20.** Séquence polynucléotidique isolée comprenant un nombre de 21 à 50 nucléotides, **caractérisée en ce qu'**une séquence selon la SEQ ID NO : 11 est contenue.

**21.** Séquence polynucléotidique isolée constituée par la SEQ ID NO : 11.

**22.** Séquence polynucléotidique isolée comprenant un nombre de 21 à 50 nucléotides, **caractérisée en ce qu'**une séquence selon la SEQ ID NO : 12 est contenue.

**23.** Séquence polynucléotidique isolée constituée par la SEQ ID NO : 12.

**24.** Utilisation d'une séquence polynucléotidique isolée selon la revendication 20 ou 21 en combinaison avec une séquence polynucléotidique isolée selon la revendication 22 ou 23 pour la multiplication d'un fragment du gène PAR 1 au moyen de la réaction PCR.

**25.** Utilisation selon la revendication 24, **caractérisée en ce que** le gène PAR 1 présente en position 3090 de la séquence selon NM_001992 un remplacement de la T par C et/ou en position 3329 de la séquence selon NM_001992 un remplacement d'A par C.

**26.** Kit contenant :

a] une séquence polynucléotidique selon la revendication 20 ou 21,
b] une séquence polynucléotidique selon la revendication 22 ou 23,
c] au moins une enzyme pour la réalisation de la réaction PCR,
d] ainsi qu'éventuellement des substances et/ou des solutions pour la réalisation de la réaction d'allongement de chaîne par polymérase (PCR),
e] ainsi qu'éventuellement d'autres séquences polynucléotidiques selon l'une ou plusieurs des revendications 1 à 17,
f] ainsi qu'éventuellement des réactifs pour la réalisation d'un séquençage.

**27.** Préparation du kit selon la revendication 26 où

a] on prépare une séquence polynucléotidique selon la revendication 20 ou 21,
b] on met à disposition une séquence polynucléotidique selon la revendication 22 ou 23,
c] on met à disposition une enzyme pour la réalisation de la réaction PCR,
d] le cas échéant des réactifs pour la réalisation d'un séquençage sont mis à disposition,
e] éventuellement, des substances et/ou des solutions pour la réalisation de la réaction d'allongement de chaîne par polymérase (PCR) sont mises à disposition,
f] éventuellement, des séquences polynucléotidiques selon l'une ou plusieurs des revendications 1 à 13 sont mises à disposition,
g] les composants de a] à e] sont introduits à chaque fois séparément dans des récipients appropriés,
h] les récipients de g] sont rassemblés, le cas échéant de manière combinée, dans une ou plusieurs unités d'emballage.

**28.** Utilisation du kit selon la revendication 26 ou 27 pour la multiplication d'un fragment du gène PAR 1 ainsi qu'éventuellement pour l'analyse ultérieure, pour détecter la présence ou non de variations dans le gène PAR 1 aux positions 3090 et/ou 3329 selon NM_001992.

Fig. 1

| | | n | % |
|---|---|---|---|
| Total | | 1404 | |
| Geschlecht | Weiblich | 406 | 28,9 |
| | Männlich | 998 | 71,1 |
| Alter* | | 62,7<br>(30,0-90,7) | |
| BMI*<br>(Body Mass Index) | | 27,8<br>(16,7-57,1) | |
| Bluthochdruck | | 834 | 59,4 |
| Raucher | | 923 | 65,7 |
| Angina pectoris | | 210 | 62,7 |
| Diabetiker (ADA) | | 445 | 31,7 |
| Herzinfarkt | | 579 | 41,0 |
| CAD (>20 %<br>Stenose) | | 1087 | 78,8 |
| Schlaganfall | | 106 | 7,5 |

*Mediane und Quartile (Q1 – Q3)

Fig. 2

| | PAR1 C3329C | PAR1 C3329A | PAR1 A3329A |
|---|---|---|---|
| PAR1 T3090T | 1 | 0 | 724 |
| PAR 1 /3090C | 0 | 371 | 160 |
| PAR1 C3090C | 51 | 47 | 8 |

Fig. 3

| | | 95 %-confidence interval | | |
|---|---|---|---|---|
| | odds ratio | lower | upper | p-value |
| Vorhofflimmern | 1,97 | 1,18 | 3,30 | 0,0101 |
| Kardiomyopathie | 1,84 | 1,04 | 3,24 | 0,0353 |

Fig. 4

| | | 95 %-confidence interval | | |
|---|---|---|---|---|
| | odds ratio | lower | upper | p-value |
| Vorhofflimmern | 2,35 | 1,18 | 4,68 | 0,0149 |
| Kardiomyopathie | 0,36 | 0,17 | 0,79 | 0,0107 |
| Instabile Angina | 0,36 | 0,16 | 0,81 | 0,0142 |

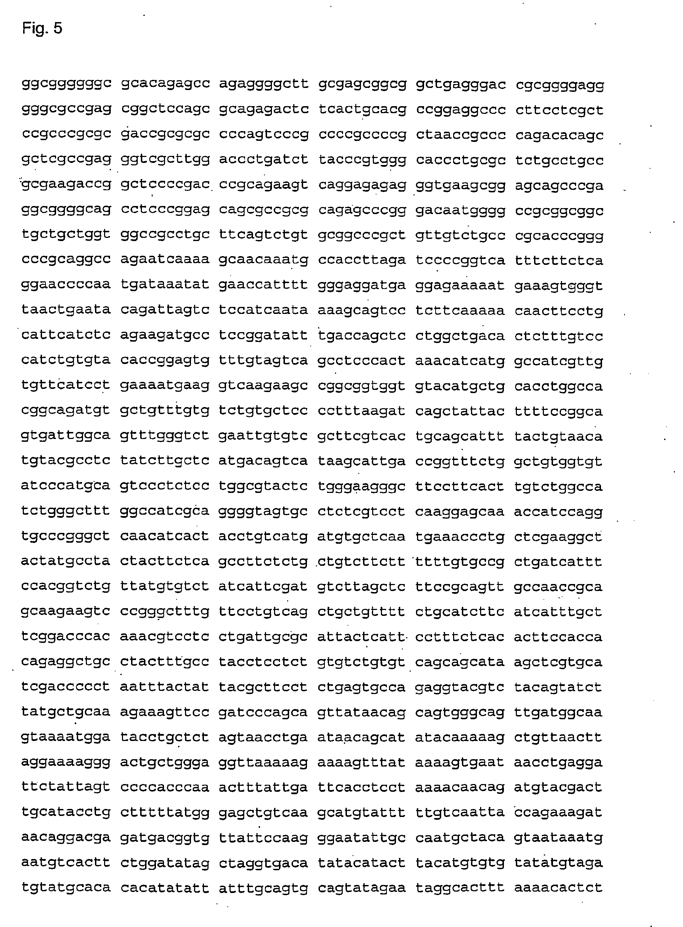

Fig. 5

ggcgggggc gcacagagcc agaggggctt gcgagcggcg gctgagggac cgcggggagg
gggcgccgag cggctccagc gcagagactc tcactgcacg ccggaggccc cttcctcgct
ccgccccgcgc gaccgcgcgc cccagtcccg ccccgccccg ctaaccgccc cagacacagc
gctcgccgag ggtcgcttgg accctgatct tacccgtggg caccctgcgc tctgcctgcc
gcgaagaccg gctccccgac ccgcagaagt caggagagag ggtgaagcgg agcagcccga
ggcggggcag cctcccggag cagcgccgcg cagagcccgg gacaatgggg ccgcggcggc
tgctgctggt ggccgcctgc ttcagtctgt gcggcccgct gttgtctgcc cgcacccggg
cccgcaggcc agaatcaaaa gcaacaaatg ccaccttaga tccccggtca tttcttctca
ggaaccccaa tgataaatat gaaccatttt gggaggatga ggagaaaaat gaaagtgggt
taactgaata cagattagtc tccatcaata aaagcagtcc tcttcaaaaa caacttcctg
cattcatctc agaagatgcc tccggatatt tgaccagctc ctggctgaca ctctttgtcc
catctgtgta caccggagtg tttgtagtca gcctcccact aaacatcatg gccatcgttg
tgttcatcct gaaaatgaag gtcaagaagc cggcggtggt gtacatgctg cacctggcca
cggcagatgt gctgtttgtg tctgtgctcc ccctttaagat cagctattac ttttccggca
gtgattggca gtttgggtct gaattgtgtc gcttcgtcac tgcagcattt tactgtaaca
tgtacgcctc tatcttgctc atgacagtca taagcattga ccggtttctg gctgtggtgt
atcccatgca gtccctctcc tggcgtactc tgggaagggc ttccttcact tgtctggcca
tctgggcttt ggccatcgca ggggtagtgc ctctcgtcct caaggagcaa accatccagg
tgcccgggct caacatcact acctgtcatg atgtgctcaa tgaaaccctg ctcgaaggct
actatgccta ctacttctca gccttctctg ctgtcttctt ttttgtgccg ctgatcattt
ccacggtctg ttatgtgtct atcattcgat gtcttagctc ttccgcagtt gccaaccgca
gcaagaagtc ccgggctttg ttcctgtcag ctgctgtttt ctgcatcttc atcatttgct
tcggacccac aaacgtcctc ctgattgcgc attactcatt cctttctcac acttccacca
cagaggctgc ctactttgcc tacctcctct gtgtctgtgt cagcagcata agctcgtgca
tcgacccccct aatttactat tacgcttcct ctgagtgcca gaggtacgtc tacagtatct
tatgctgcaa agaaagttcc gatcccagca gttataacag cagtgggcag ttgatggcaa
gtaaaatgga tacctgctct agtaacctga ataacagcat atacaaaaag ctgttaactt
aggaaaaggg actgctggga ggttaaaaag aaaagtttat aaaagtgaat aacctgagga
ttctattagt ccccacccaa actttattga ttcacctcct aaaacaacag atgtacgact
tgcatacctg ctttttatgg gagctgtcaa gcatgtattt ttgtcaatta ccagaaagat
aacaggacga gatgacggtg ttattccaag ggaatattgc caatgctaca gtaataaatg
aatgtcactt ctggatatag ctaggtgaca tatacatact tacatgtgtg tatatgtaga
tgtatgcaca cacatatatt atttgcagtg cagtatagaa taggcacttt aaaacactct

ttccccgcac cccagcaatt atgaaaataa tctctgattc cctgatttaa tatgcaaagt
ctaggttggt agagtttagc cctgaacatt tcatggtgtt catcaacagt gagagactcc
atagtttggg cttgtaccac ttttgcaaat aagtgtattt tgaaattgtt tgacggcaag
gtttaagtta ttaagaggta agacttagta ctatctgtgc gtagaagttc tagtgttttc
aattttaaac atatccaagt ttgaattcct aaaattatgg aaacagatga aaagcctctg
ttttgatatg ggtagtattt tttacatttt acacactgta cacataagcc aaaactgagc
ataagtcctc tagtgaatgt aggctggctt tcagagtagg ctattcctga gagctgcatg
tgtccgcccc cgatggagga ctccaggcag cagacacatg ccagggccat gtcagacaca
gattggccag aaaccttcct gctgagcctc acagcagtga gactggggcc actacatttg
ctccatcctc ctgggattgg ctgtgaactg atcatgttta tgagaaactg gcaaagcaga
atgtgatatc ctaggaggta atgaccatga aagacttctc tacccatctt aaaaacaacg
aaagaaggca tggacttctg gatgcccatc cactgggtgt aaacacatct agtagttgtt
ctgaaatgtc agttctgata tggaagcacc cattatgcgc tgtggccact ccaataggtg
ctgagtgtac agagtggaat aagacagaga cctgccctca agagcaaagt agatcatgca
tagagtgtga tgtatgtgta ataaatatgt ttcacacaaa caaggcctgt cagctaaaga
agtttgaaca tttgggttac tatttcttgt ggttataact taatgaaaac aatgcagtac
aggacatata tttttaaaa taagtctgat ttaattgggc actatttatt tacaaatgtt
ttgctcaata gattgctcaa atcaggtttt cttttaagaa tcaatcatgt cagtctgctt
agaaataaca gaagaaaata gaattgacat tgaaatctag gaaaattatt ctataatttc
catttactta agacttaatg agactttaaa agcatttttt aacctcctaa gtatcaagta
tagaaaatct tcatggaatt cacaaagtaa tttggaaatt aggttgaaac atatctctta
tcttacgaaa aaatggtagc attttaaaca aaatagaaag ttgcaaggca aatgtttatt
taaaagagca ggccaggcgc ggtggctcac gcctgtaatc ccagcacttt gggaggctga
ggcgggtgga tcacgaggtc aggagatcga gaccatcctg gctaacacgg tgaaacccgt
ctctactaaa aatgcaaaaa aaattagccg ggcgtggtgg caggcacctg tagtcccagc
tactcgggag gctgaggcag gagactggcg tgaacccagg aggcggacct tgtagtgagc
cgagatcgcg ccactgtgct ccagcctggg caacagagca agactccatc tc

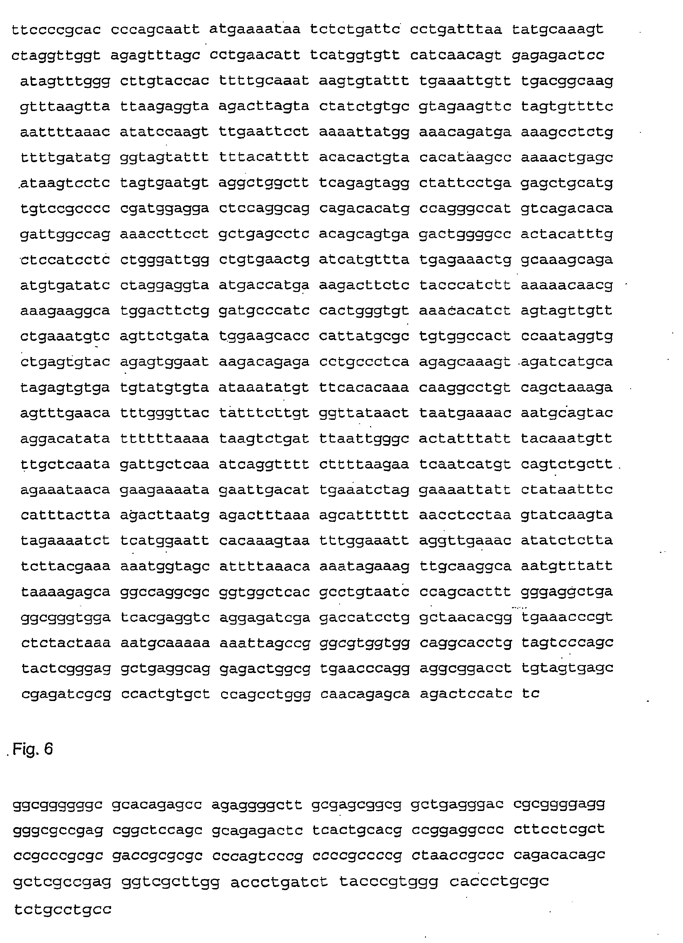

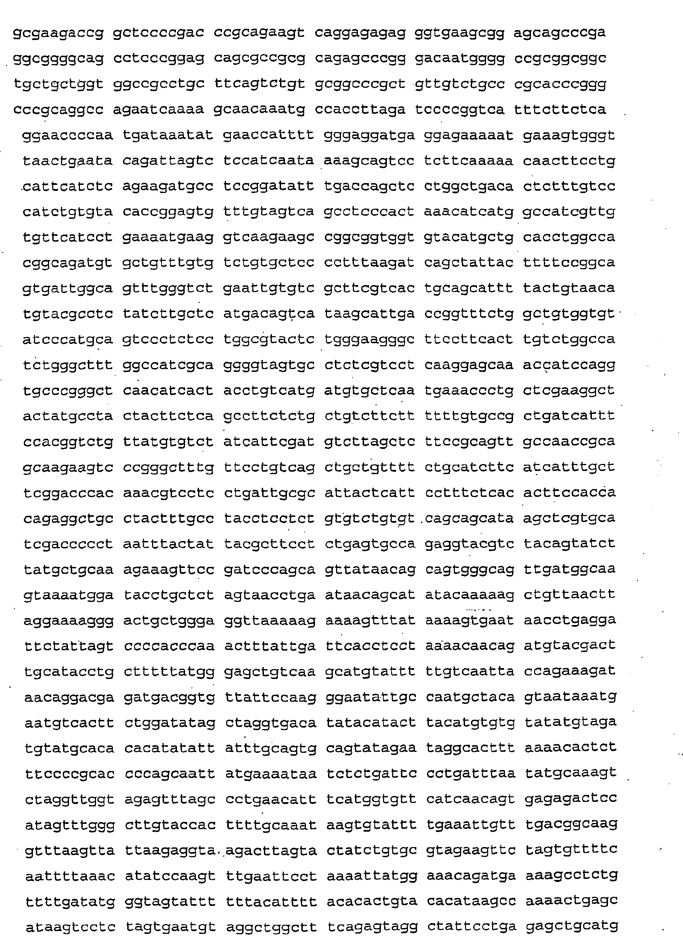

Fig. 6

ggcgggggc gcacagagcc agaggggctt gcgagcggcg gctgagggac cgcggggagg
gggcgccgag cggctccagc gcagagactc tcactgcacg ccggaggccc cttcctcgct
ccgcccgcgc gaccgcgcgc cccagtcccg ccccgccccg ctaaccgccc cagacacagc
gctcgccgag ggtcgcttgg accctgatct tacccgtggg caccctgcgc
tctgcctgcc

gcgaagaccg gctccccgac ccgcagaagt caggagagag ggtgaagcgg agcagcccga
ggcggggcag cctcccggag cagcgccgcg cagagcccgg gacaatgggg ccgcggcggc
tgctgctggt ggccgcctgc ttcagtctgt gcggcccgct gttgtctgcc cgcacccggg
cccgcaggcc agaatcaaaa gcaacaaatg ccaccttaga tccccggtca tttcttctca
ggaaccccaa tgataaatat gaaccatttt gggaggatga ggagaaaaat gaaagtgggt
taactgaata cagattagtc tccatcaata aaagcagtcc tcttcaaaaa caacttcctg
cattcatctc agaagatgcc tccggatatt tgaccagctc ctggctgaca ctctttgtcc
catctgtgta caccggagtg tttgtagtca gcctcccact aaacatcatg gccatcgttg
tgttcatcct gaaaatgaag gtcaagaagc cggcggtggt gtacatgctg cacctggcca
cggcagatgt gctgtttgtg tctgtgctcc cctttaagat cagctattac ttttccggca
gtgattggca gtttgggtct gaattgtgtc gcttcgtcac tgcagcattt tactgtaaca
tgtacgcctc tatcttgctc atgacagtca taagcattga ccggtttctg gctgtggtgt
atcccatgca gtccctctcc tggcgtactc tgggaagggc ttccttcact tgtctggcca
tctgggcttt ggccatcgca ggggtagtgc ctctcgtcct caaggagcaa accatccagg
tgcccgggct caacatcact acctgtcatg atgtgctcaa tgaaaccctg ctcgaaggct
actatgccta ctacttctca gccttctctg ctgtcttctt ttttgtgccg ctgatcattt
ccacggtctg ttatgtgtct atcattcgat gtcttagctc ttccgcagtt gccaaccgca
gcaagaagtc ccgggctttg ttcctgtcag ctgctgtttt ctgcatcttc atcatttgct
tcggacccac aaacgtcctc ctgattgcgc attactcatt cctttctcac acttccacca
cagaggctgc ctactttgcc tacctcctct gtgtctgtgt cagcagcata agctcgtgca
tcgacccccct aatttactat tacgcttcct ctgagtgcca gaggtacgtc tacagtatct
tatgctgcaa agaaagttcc gatcccagca gttataacag cagtgggcag ttgatggcaa
gtaaaatgga tacctgctct agtaacctga ataacagcat atacaaaaag ctgttaactt
aggaaaaggg actgctggga ggttaaaaag aaaagtttat aaaagtgaat aacctgagga
ttctattagt ccccacccaa actttattga ttcacctcct aaaacaacag atgtacgact
tgcatacctg ctttttatgg gagctgtcaa gcatgtattt ttgtcaatta ccagaaagat
aacaggacga gatgacggtg ttattccaag ggaatattgc caatgctaca gtaataaatg
aatgtcactt ctggatatag ctaggtgaca tatacatact tacatgtgtg tatatgtaga
tgtatgcaca cacatatatt atttgcagtg cagtatagaa taggcacttt aaaacactct
ttccccgcac cccagcaatt atgaaaataa tctctgattc cctgatttaa tatgcaaagt
ctaggttggt agagtttagc cctgaacatt tcatggtgtt catcaacagt gagagactcc
atagtttggg cttgtaccac ttttgcaaat aagtgtattt tgaaattgtt tgacggcaag
gtttaagtta ttaagaggta agacttagta ctatctgtgc gtagaagttc tagtgttttc
aattttaaac atatccaagt ttgaattcct aaaattatgg aaacagatga aaagcctctg
ttttgatatg ggtagtattt tttacatttt acacactgta cacataagcc aaaactgagc
ataagtcctc tagtgaatgt aggctggctt tcagagtagg ctattcctga gagctgcatg

tgtccgcccc cgatggagga ctccaggcag cagacacatg ccagggccat gtcagacaca
gattggccag aaaccttcct gctgagcctc acagcagtga gactggggcc actacatttg
ctccatcctc ctgggattgg ctgtgaactg atcatgttta tgagaaactg gcaaagcaga
atgtgatatc ctaggaggta atgaccatga aagacttctc tacccatctt aaaaacaacg
aaagaaggca tggacttctg gatgcccatc cactgggtgt aaacacatct agtagttgtt
ctgaaatgtc agttctgata tggaagcacc cattatgcgc tgtggccact ccaataggtg
ctgagtgtac agagtggaat aagacagaga cctgccctca agagcaaagt agatcatgca
tagagtgtga tgtatgtgta ataaatatgt ttcacacaaa caaggcctgt cagctaaaga
agtttgaaca tttgggttac tatttcttgt ggttataact taatgaaaac aatgcagtac
aggacatata tttttaaaa taagtctgat ttaattgggc actatttatt tacaaatgtt
ttgctcaata gattgctcaa atcaggtttt cttttaagaa tcaatcatgt cagtctgctt
agaaataaca gaagaaaata gaattgacac tgaaatctag gaaaattatt ctataatttc
catttactta agacttaatg agactttaaa agcatttttt aacctcctaa gtatcaagta
tagaaaatct tcatggaatt cacaaagtaa tttggaaatt aggttgaaac atatctctta
tcttacgaaa aaatggtagc attttaaaca aaatagaaag ttgcaaggca aatgtttatt
taaaagagca ggccaggcgc ggtggctcac gcctgtaatc ccagcacttt gggaggctga
ggcgggtgga tcacgaggtc aggagatcga gaccatcctg gctaacacgg tgaaacccgt
ctctactaaa aatgcaaaaa aaattagccg ggcgtggtgg caggcacctg tagtcccagc
tactcgggag gctgaggcag gagactggcg tgaacccagg aggcggacct tgtagtgagc
cgagatcgcg ccactgtgct ccagcctggg caacagagca agactccatc tc

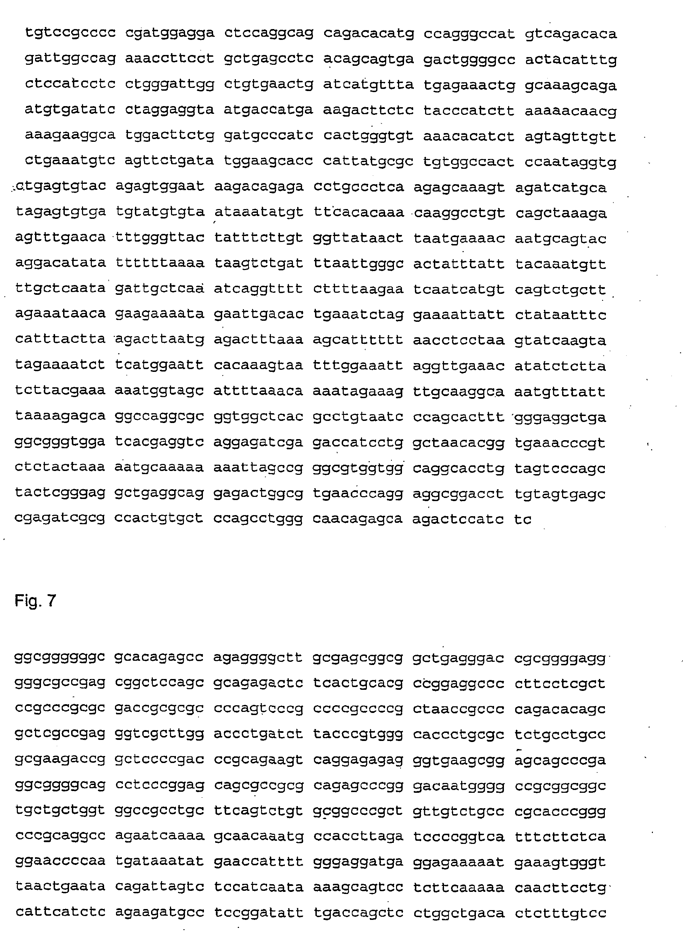

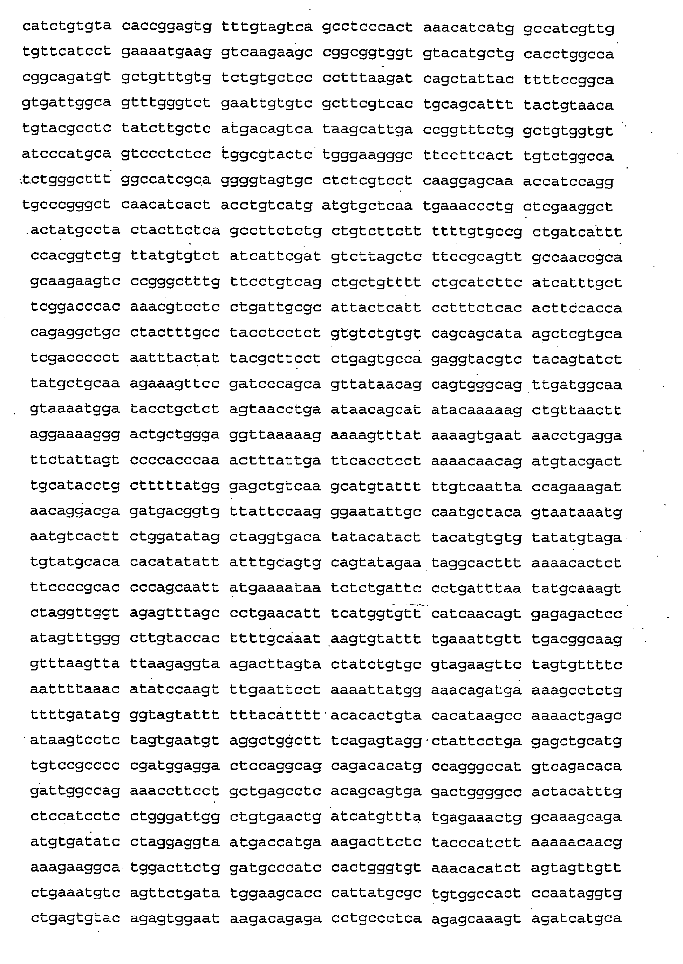

Fig. 7

ggcgggggc gcacagagcc agaggggctt gcgagcggcg gctgagggac cgcggggagg
gggcgccgag cggctccagc gcagagactc tcactgcacg ccggaggccc cttcctcgct
ccgcccgcgc gaccgcgcgc cccagtcccg ccccgccccg ctaaccgccc cagacacagc
gctcgccgag ggtcgcttgg accctgatct tacccgtggg caccctgcgc tctgcctgcc
gcgaagaccg gctccccgac ccgcagaagt caggagagag ggtgaagcgg agcagcccga
ggcggggcag cctcccggag cagcgccgcg cagagcccgg gacaatgggg ccgcggcggc
tgctgctggt ggccgcctgc ttcagtctgt gcggcccgct gttgtctgcc cgcacccggg
cccgcaggcc agaatcaaaa gcaacaaatg ccaccttaga tccccggtca tttcttctca
ggaaccccaa tgataaatat gaaccatttt gggaggatga ggagaaaaat gaaagtgggt
taactgaata cagattagtc tccatcaata aaagcagtcc tcttcaaaaa caacttcctg
cattcatctc agaagatgcc tccggatatt tgaccagctc ctggctgaca ctctttgtcc

catctgtgta caccggagtg tttgtagtca gcctcccact aaacatcatg gccatcgttg
tgttcatcct gaaaatgaag gtcaagaagc cggcggtggt gtacatgctg cacctggcca
cggcagatgt gctgtttgtg tctgtgctcc cctttaagat cagctattac ttttccggca
gtgattggca gtttgggtct gaattgtgtc gcttcgtcac tgcagcattt tactgtaaca
tgtacgcctc tatcttgctc atgacagtca taagcattga ccggtttctg gctgtggtgt
atcccatgca gtccctctcc tggcgtactc tgggaagggc ttccttcact tgtctggcca
tctgggcttt ggccatcgca ggggtagtgc ctctcgtcct caaggagcaa accatccagg
tgcccgggct caacatcact acctgtcatg atgtgctcaa tgaaaccctg ctcgaaggct
actatgccta ctacttctca gccttctctg ctgtcttctt ttttgtgccg ctgatcattt
ccacggtctg ttatgtgtct atcattcgat gtcttagctc ttccgcagtt gccaaccgca
gcaagaagtc ccgggctttg ttcctgtcag ctgctgtttt ctgcatcttc atcatttgct
tcggacccac aaacgtcctc ctgattgcgc attactcatt cctttctcac acttccacca
cagaggctgc ctactttgcc tacctcctct gtgtctgtgt cagcagcata agctcgtgca
tcgaccccct aatttactat tacgcttcct ctgagtgcca gaggtacgtc tacagtatct
tatgctgcaa agaaagttcc gatcccagca gttataacag cagtgggcag ttgatggcaa
gtaaaatgga tacctgctct agtaacctga ataacagcat atacaaaaag ctgttaactt
aggaaaaggg actgctggga ggttaaaaag aaaagtttat aaaagtgaat aacctgagga
ttctattagt ccccacccaa actttattga ttcacctcct aaaacaacag atgtacgact
tgcatacctg ctttttatgg gagctgtcaa gcatgtattt ttgtcaatta ccagaaagat
aacaggacga gatgacggtg ttattccaag ggaatattgc caatgctaca gtaataaatg
aatgtcactt ctggatatag ctaggtgaca tatacatact tacatgtgtg tatatgtaga
tgtatgcaca cacatatatt atttgcagtg cagtatagaa taggcacttt aaaacactct
ttccccgcac cccagcaatt atgaaaataa tctctgattc cctgatttaa tatgcaaagt
ctaggttggt agagtttagc cctgaacatt tcatggtgtt catcaacagt gagagactcc
atagtttggg cttgtaccac ttttgcaaat aagtgtattt tgaaattgtt tgacggcaag
gtttaagtta ttaagaggta agacttagta ctatctgtgc gtagaagttc tagtgttttc
aattttaaac atatccaagt ttgaattcct aaaattatgg aaacagatga aaagcctctg
ttttgatatg ggtagtattt tttacatttt acacactgta cacataagcc aaaactgagc
ataagtcctc tagtgaatgt aggctggctt tcagagtagg ctattcctga gagctgcatg
tgtccgcccc cgatggagga ctccaggcag cagacacatg ccagggccat gtcagacaca
gattggccag aaaccttcct gctgagcctc acagcagtga gactggggcc actacatttg
ctccatcctc ctgggattgg ctgtgaactg atcatgttta tgagaaactg gcaaagcaga
atgtgatatc ctaggaggta atgaccatga aagacttctc tacccatctt aaaaacaacg
aaagaaggca tggacttctg gatgcccatc cactgggtgt aaacacatct agtagttgtt
ctgaaatgtc agttctgata tggaagcacc cattatgcgc tgtggccact ccaataggtg
ctgagtgtac agagtggaat aagacagaga cctgccctca agagcaaagt agatcatgca

tagagtgtga tgtatgtgta ataaatatgt ttcacacaaa caaggcctgt cagctaaaga
agtttgaaca tttgggttac tatttcttgt ggttataact taatgaaaac aatgcagtac
aggacatata ttttttaaaa taagtctgat ttaattgggc actatttatt tacaaatgtt
ttgctcaata gattgctcaa atcaggtttt cttttaagaa tcaatcatgt cagtctgctt
agaaataaca gaagaaaata gaattgacat tgaaatctag gaaaattatt ctataatttc
catttactta agacttaatg agactttaaa agcatttttt aacctcctaa gtatcaagta
tagaaaatct tcatggaatt cacaaagtaa tttggaaatt aggttgaaac atatctctta
tcttacgaaa aaatggtagc attttaaaca aaatagaaag ttgcaaggca aatgtttatt
taaaagagca ggccaggcgc ggtggctccc gcctgtaatc ccagcacttt gggaggctga
ggcgggtgga tcacgaggtc aggagatcga gaccatcctg gctaacacgg tgaaacccgt
ctctactaaa aatgcaaaaa aaattagccg ggcgtggtgg caggcacctg tagtcccagc
tactcgggag gctgaggcag gagactggcg tgaacccagg aggcggacct tgtagtgagc
cgagatcgcg ccactgtgct ccagcctggg caacagagca agactccatc tc

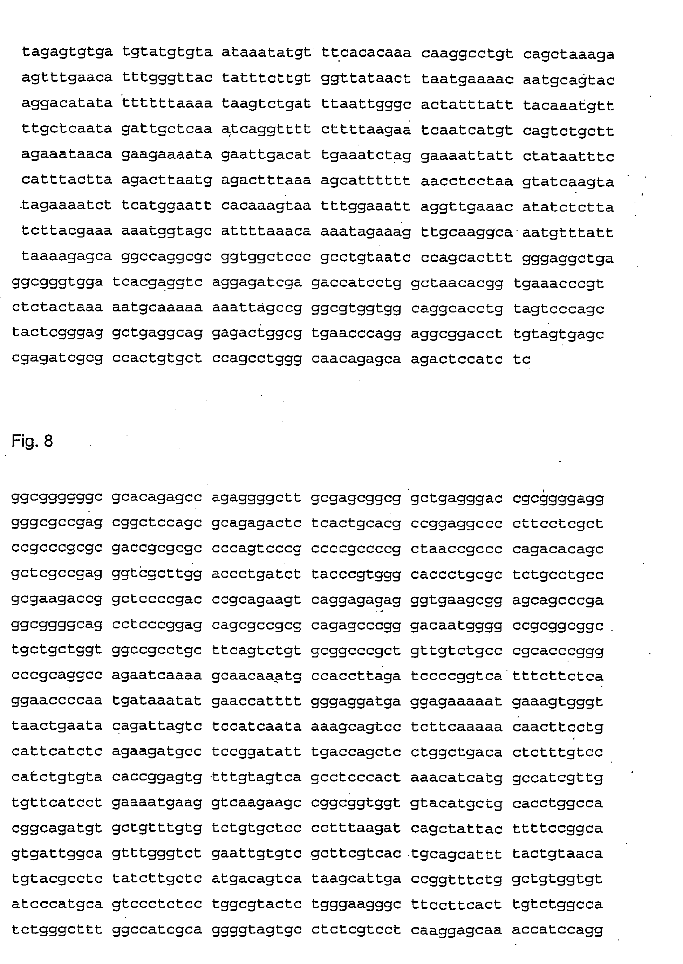

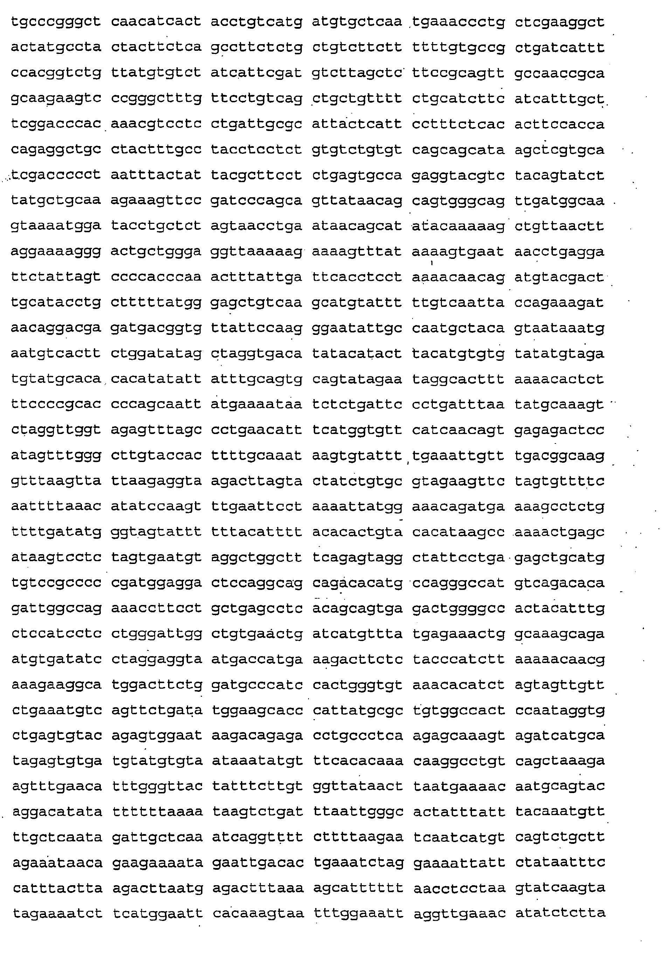

Fig. 8

ggcggggggc gcacagagcc agaggggctt gcgagcggcg gctgagggac cgcggggagg
gggcgccgag cggctccagc gcagagactc tcactgcacg ccggaggccc cttcctcgct
ccgcccgcgc gaccgcgcgc cccagtcccg ccccgccccg ctaaccgccc cagacacagc
gctcgccgag ggtcgcttgg accctgatct tacccgtggg caccctgcgc tctgcctgcc
gcgaagaccg gctccccgac ccgcagaagt caggagagag ggtgaagcgg agcagcccga
ggcggggcag cctcccggag cagcgccgcg cagagcccgg gacaatgggg ccgcggcggc
tgctgctggt ggccgcctgc ttcagtctgt gcggcccgct gttgtctgcc cgcacccggg
cccgcaggcc agaatcaaaa gcaacaaatg ccaccttaga tccccggtca tttcttctca
ggaaccccaa tgataaatat gaaccatttt gggaggatga ggagaaaaat gaaagtgggt
taactgaata cagattagtc tccatcaata aaagcagtcc tcttcaaaaa caacttcctg
cattcatctc agaagatgcc tccggatatt tgaccagctc ctggctgaca ctctttgtcc
catctgtgta caccggagtg tttgtagtca gcctcccact aaacatcatg gccatcgttg
tgttcatcct gaaaatgaag gtcaagaagc cggcggtggt gtacatgctg cacctggcca
cggcagatgt gctgtttgtg tctgtgctcc cctttaagat cagctattac ttttccggca
gtgattggca gtttgggtct gaattgtgtc gcttcgtcac tgcagcattt tactgtaaca
tgtacgcctc tatcttgctc atgacagtca taagcattga ccggtttctg gctgtggtgt
atcccatgca gtccctctcc tggcgtactc tgggaagggc ttccttcact tgtctggcca
tctgggcttt ggccatcgca ggggtagtgc ctctcgtcct caaggagcaa accatccagg

tgcccgggct caacatcact acctgtcatg atgtgctcaa tgaaaccctg ctcgaaggct
actatgccta ctacttctca gccttctctg ctgtcttctt ttttgtgccg ctgatcattt
ccacggtctg ttatgtgtct atcattcgat gtcttagctc ttccgcagtt gccaaccgca
gcaagaagtc ccgggctttg ttcctgtcag ctgctgtttt ctgcatcttc atcatttgct
tcggacccac aaacgtcctc ctgattgcgc attactcatt cctttctcac acttccacca
cagaggctgc ctactttgcc tacctcctct gtgtctgtgt cagcagcata agctcgtgca
tcgacccccct aatttactat tacgcttcct ctgagtgcca gaggtacgtc tacagtatct
tatgctgcaa agaaagttcc gatcccagca gttataacag cagtgggcag ttgatggcaa
gtaaaatgga tacctgctct agtaacctga ataacagcat atacaaaaag ctgttaactt
aggaaaaggg actgctggga ggttaaaaag aaaagtttat aaaagtgaat aacctgagga
ttctattagt ccccacccaa actttattga ttcacctcct aaaacaacag atgtacgact
tgcatacctg ctttttatgg gagctgtcaa gcatgtattt ttgtcaatta ccagaaagat
aacaggacga gatgacggtg ttattccaag ggaatattgc caatgctaca gtaataaatg
aatgtcactt ctggatatag ctaggtgaca tatacatact tacatgtgtg tatatgtaga
tgtatgcaca cacatatatt atttgcagtg cagtatagaa taggcacttt aaaacactct
ttccccgcac cccagcaatt atgaaaataa tctctgattc cctgatttaa tatgcaaagt
ctaggttggt agagtttagc cctgaacatt tcatggtgtt catcaacagt gagagactcc
atagtttggg cttgtaccac ttttgcaaat aagtgtattt tgaaattgtt tgacggcaag
gtttaagtta ttaagaggta agacttagta ctatctgtgc gtagaagttc tagtgttttc
aattttaaac atatccaagt ttgaattcct aaaattatgg aaacagatga aaagcctctg
ttttgatatg ggtagtattt tttacatttt acacactgta cacataagcc aaaactgagc
ataagtcctc tagtgaatgt aggctggctt tcagagtagg ctattcctga gagctgcatg
tgtccgcccc cgatggagga ctccaggcag cagacacatg ccagggccat gtcagacaca
gattggccag aaaccttcct gctgagcctc acagcagtga gactggggcc actacatttg
ctccatcctc ctgggattgg ctgtgaactg atcatgttta tgagaaactg gcaaagcaga
atgtgatatc ctaggaggta atgaccatga aagacttctc tacccatctt aaaaacaacg
aaagaaggca tggacttctg gatgcccatc cactgggtgt aaacacatct agtagttgtt
ctgaaatgtc agttctgata tggaagcacc cattatgcgc tgtggccact ccaataggtg
ctgagtgtac agagtggaat aagacagaga cctgccctca agagcaaagt agatcatgca
tagagtgtga tgtatgtgta ataaatatgt ttcacacaaa caaggcctgt cagctaaaga
agtttgaaca tttgggttac tatttcttgt ggttataact taatgaaaac aatgcagtac
aggacatata ttttttaaaa taagtctgat ttaattgggc actatttatt tacaaatgtt
ttgctcaata gattgctcaa atcaggtttt cttttaagaa tcaatcatgt cagtctgctt
agaaataaca gaagaaaata gaattgacac tgaaatctag gaaaattatt ctataatttc
catttactta agacttaatg agactttaaa agcatttttt aacctcctaa gtatcaagta
tagaaaatct tcatggaatt cacaaagtaa tttggaaatt aggttgaaac atatctctta

tcttacgaaa aaatggtagc attttaaaca aaatagaaag ttgcaaggca aatgtttatt
taaaagagca ggccaggcgc ggtggctccc gcctgtaatc ccagcacttt gggaggctga
ggcgggtgga tcacgaggtc aggagatcga gaccatcctg gctaacacgg tgaaaccccgt
ctctactaaa aatgcaaaaa aaattagccg ggcgtggtgg caggcacctg tagtcccagc
tactcgggag gctgaggcag gagactggcg tgaacccagg aggcggacct tgtagtgagc
cgagatcgcg ccactgtgct ccagcctggg caacagagca agactccatc tc

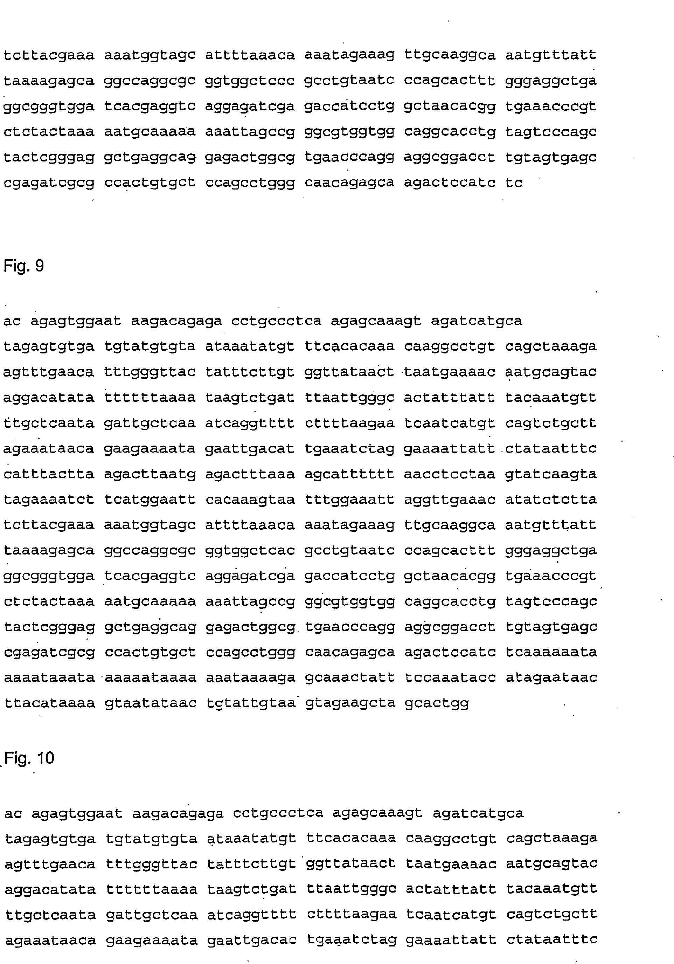

Fig. 9

ac agagtggaat aagacagaga cctgccctca agagcaaagt agatcatgca
tagagtgtga tgtatgtgta ataaatatgt ttcacacaaa caaggcctgt cagctaaaga
agtttgaaca tttgggttac tatttcttgt ggttataact taatgaaaac aatgcagtac
aggacatata ttttttaaaa taagtctgat ttaattgggc actatttatt tacaaatgtt
ttgctcaata gattgctcaa atcaggtttt cttttaagaa tcaatcatgt cagtctgctt
agaaataaca gaagaaaata gaattgacat tgaaatctag gaaaattatt ctataatttc
catttactta agacttaatg agactttaaa agcatttttt aacctcctaa gtatcaagta
tagaaaatct tcatggaatt cacaaagtaa tttggaaatt aggttgaaac atatctctta
tcttacgaaa aaatggtagc attttaaaca aaatagaaag ttgcaaggca aatgtttatt
taaaagagca ggccaggcgc ggtggctcac gcctgtaatc ccagcacttt gggaggctga
ggcgggtgga tcacgaggtc aggagatcga gaccatcctg gctaacacgg tgaaaccccgt
ctctactaaa aatgcaaaaa aaattagccg ggcgtggtgg caggcacctg tagtcccagc
tactcgggag gctgaggcag gagactggcg tgaacccagg aggcggacct tgtagtgagc
cgagatcgcg ccactgtgct ccagcctggg caacagagca agactccatc tcaaaaaata
aaaataaata aaaaataaaa aaataaaaga gcaaactatt tccaaatacc atagaataac
ttacataaaa gtaatataac tgtattgtaa gtagaagcta gcactgg

Fig. 10

ac agagtggaat aagacagaga cctgccctca agagcaaagt agatcatgca
tagagtgtga tgtatgtgta ataaatatgt ttcacacaaa caaggcctgt cagctaaaga
agtttgaaca tttgggttac tatttcttgt ggttataact taatgaaaac aatgcagtac
aggacatata ttttttaaaa taagtctgat ttaattgggc actatttatt tacaaatgtt
ttgctcaata gattgctcaa atcaggtttt cttttaagaa tcaatcatgt cagtctgctt
agaaataaca gaagaaaata gaattgacac tgaaatctag gaaaattatt ctataatttc

catttactta agacttaatg agactttaaa agcatttttt aacctcctaa gtatcaagta
tagaaaatct tcatggaatt cacaaagtaa tttggaaatt aggttgaaac atatctctta
tcttacgaaa aaatggtagc attttaaaca aaatagaaag ttgcaaggca aatgtttatt
taaaagagca ggccaggcgc ggtggctcac gcctgtaatc ccagcacttt gggaggctga
ggcgggtgga tcacgaggtc aggagatcga gaccatcctg gctaacacgg tgaaacccgt
ctctactaaa aatgcaaaaa aaattagccg ggcgtggtgg caggcacctg tagtcccagc
tactcgggag gctgaggcag gagactggcg tgaacccagg aggcggacct tgtagtgagc
cgagatcgcg ccactgtgct ccagcctggg caacagagca agactccatc tcaaaaaata
aaaataaata aaaaataaaa aaataaaaga gcaaactatt tccaaatacc atagaataac
ttacataaaa gtaatataac tgtattgtaa gtagaagcta gcactgg

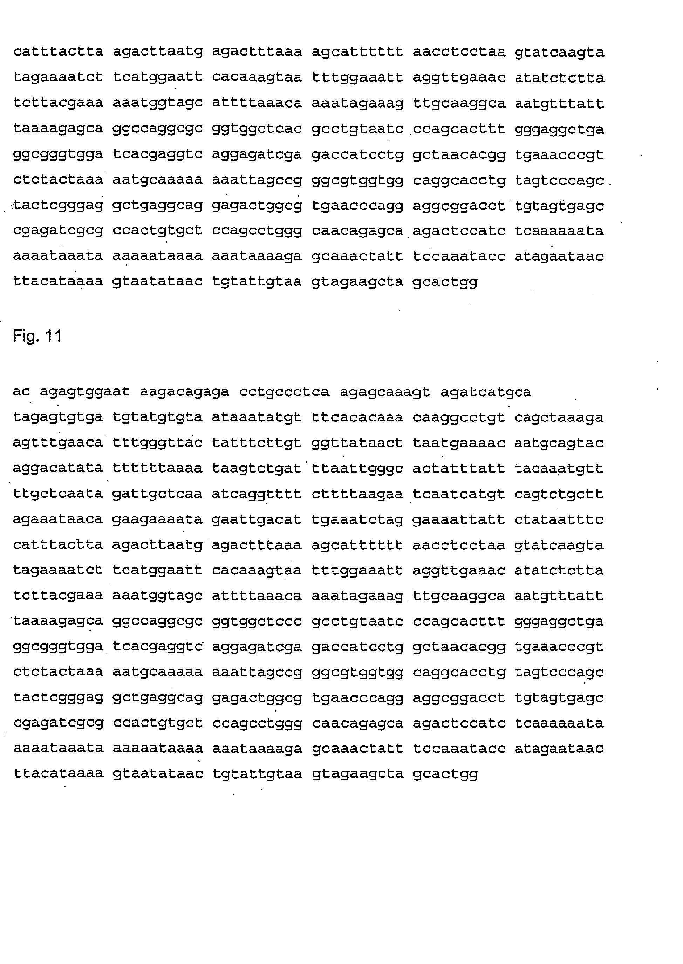

Fig. 11

ac agagtggaat aagacagaga cctgccctca agagcaaagt agatcatgca
tagagtgtga tgtatgtgta ataaatatgt ttcacacaaa caaggcctgt cagctaaaga
agtttgaaca tttgggttac tatttcttgt ggttataact taatgaaaac aatgcagtac
aggacatata tttttaaaa taagtctgat ttaattgggc actatttatt tacaaatgtt
ttgctcaata gattgctcaa atcaggtttt cttttaagaa tcaatcatgt cagtctgctt
agaaataaca gaagaaaata gaattgacat tgaaatctag gaaaattatt ctataatttc
catttactta agacttaatg agactttaaa agcatttttt aacctcctaa gtatcaagta
tagaaaatct tcatggaatt cacaaagtaa tttggaaatt aggttgaaac atatctctta
tcttacgaaa aaatggtagc attttaaaca aaatagaaag ttgcaaggca aatgtttatt
taaaagagca ggccaggcgc ggtggctccc gcctgtaatc ccagcacttt gggaggctga
ggcgggtgga tcacgaggtc aggagatcga gaccatcctg gctaacacgg tgaaacccgt
ctctactaaa aatgcaaaaa aaattagccg ggcgtggtgg caggcacctg tagtcccagc
tactcgggag gctgaggcag gagactggcg tgaacccagg aggcggacct tgtagtgagc
cgagatcgcg ccactgtgct ccagcctggg caacagagca agactccatc tcaaaaaata
aaaataaata aaaaataaaa aaataaaaga gcaaactatt tccaaatacc atagaataac
ttacataaaa gtaatataac tgtattgtaa gtagaagcta gcactgg

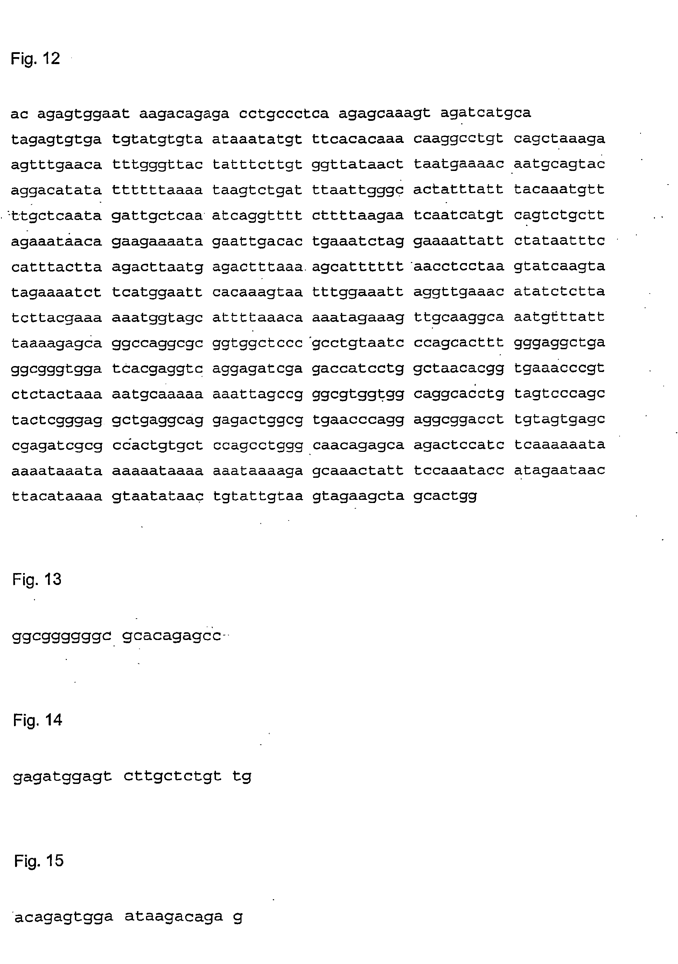

Fig. 12

ac agagtggaat aagacagaga cctgccctca agagcaaagt agatcatgca
tagagtgtga tgtatgtgta ataaatatgt ttcacacaaa caaggcctgt cagctaaaga
agtttgaaca tttgggttac tatttcttgt ggttataact taatgaaaac aatgcagtac
aggacatata ttttttaaaa taagtctgat ttaattgggc actatttatt tacaaatgtt
ttgctcaata gattgctcaa atcaggtttt cttttaagaa tcaatcatgt cagtctgctt
agaaataaca gaagaaaata gaattgacac tgaaatctag gaaaattatt ctataatttc
catttactta agacttaatg agactttaaa agcatttttt aacctcctaa gtatcaagta
tagaaaatct tcatggaatt cacaaagtaa tttggaaatt aggttgaaac atatctctta
tcttacgaaa aaatggtagc attttaaaca aaatagaaag ttgcaaggca aatgtttatt
taaaagagca ggccaggcgc ggtggctccc gcctgtaatc ccagcacttt gggaggctga
ggcgggtgga tcacgaggtc aggagatcga gaccatcctg gctaacacgg tgaaacccgt
ctctactaaa aatgcaaaaa aaattagccg ggcgtggtgg caggcacctg tagtcccagc
tactcgggag gctgaggcag gagactggcg tgaacccagg aggcggacct tgtagtgagc
cgagatcgcg ccactgtgct ccagcctggg caacagagca agactccatc tcaaaaaata
aaaataaata aaaaataaaa aaataaaaga gcaaactatt tccaaatacc atagaataac
ttacataaaa gtaatataac tgtattgtaa gtagaagcta gcactgg

Fig. 13

ggcgggggc gcacagagcc

Fig. 14

gagatggagt cttgctctgt tg

Fig. 15

acagagtgga ataagacaga g

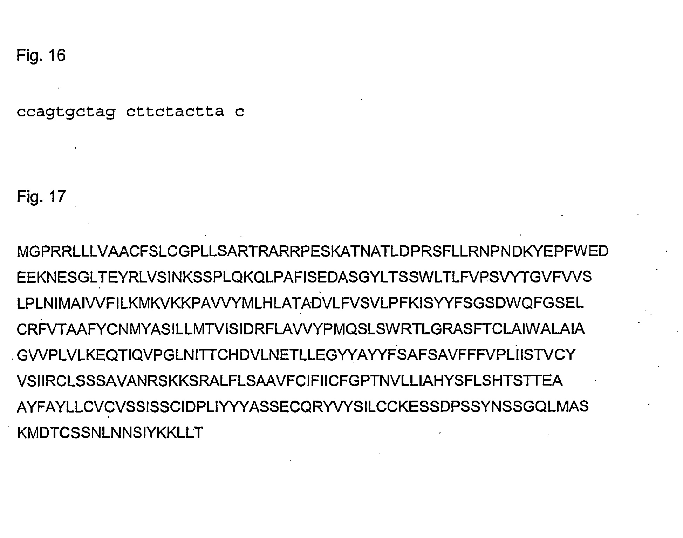

Fig. 16

```
ccagtgctag cttctactta c
```

Fig. 17

MGPRRLLLVAACFSLCGPLLSARTRARRPESKATNATLDPRSFLLRNPNDKYEPFWED
EEKNESGLTEYRLVSINKSSPLQKQLPAFISEDASGYLTSSWLTLFVPSVYTGVFVVS
LPLNIMAIVVFILKMKVKKPAVVYMLHLATADVLFVSVLPFKISYYFSGSDWQFGSEL
CRFVTAAFYCNMYASILLMTVISIDRFLAVVYPMQSLSWRTLGRASFTCLAIWALAIA
GVVPLVLKEQTIQVPGLNITTCHDVLNETLLEGYYAYYFSAFSAVFFFVPLIISTVCY
VSIIRCLSSSAVANRSKKSRALFLSAAVFCIFIICFGPTNVLLIAHYSFLSHTSTTEA
AYFAYLLCVCVSSISSCIDPLIYYYASSECQRYVYSILCCKESSDPSSYNSSGQLMAS
KMDTCSSNLNNSIYKKLLT

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Nicht-Patentliteratur


- **SCHMIDT et al.** *J. Biol. Chem.,* 1996, vol. 271, 9307-9312
- Current Protocols in Molecular Biology. John Wiley & Sons, Inc,
- Short Protocols in Molecular Biology. John Wiley & Sons, Inc, Oktober 2002
- **J. SAMBROCK ; E.F. FRITSCH ; T. MANIATIS.** Molecular Cloning. Cold Spring Harbor Laboratory Press

SEQUENCE LISTING

<110> Aventis Pharma Deutschland GmbH

<120> Analyse und Verwendung von PAR 1 Polymorphismen zur Risikoabschätzung für kardiovaskuläre Erkankungen

<130> DEAV 2003/0030

<140>
<141>

<160> 13

<170> PatentIn Ver. 2.1

<210> 1
<211> 3592
<212> DNA
<213> Homo sapiens

<400> 1

```
ggcgggggc gcacagagcc agaggggctt gcgagcggcg gctgagggac cgcggggagg 60
gggcgccgag cggctccagc gcagagactc tcactgcacg ccggaggccc cttcctcgct 120
ccgcccgcgc gaccgcgcgc cccagtcccg ccccgccccg ctaaccgccc cagacacagc 180
gctcgccgag ggtcgcttgg accctgatct tacccgtggg caccctgcgc tctgcctgcc 240
gcgaagaccg gctccccgac ccgcagaagt caggagagag ggtgaagcgg agcagcccga 300
ggcggggcag cctcccggag cagcgccgcg cagagcccgg gacaatgggg ccgcggcggc 360
tgctgctggt ggccgcctgc ttcagtctgt gcggcccgct gttgtctgcc cgcacccggg 420
cccgcaggcc agaatcaaaa gcaacaaatg ccaccttaga tccccggtca tttcttctca 480
ggaaccccaa tgataaatat gaaccatttt gggaggatga ggagaaaaat gaaagtgggt 540
taactgaata cagattagtc tccatcaata aaagcagtcc tcttcaaaaa caacttcctg 600
cattcatctc agaagatgcc tccggatatt tgaccagctc ctggctgaca ctctttgtcc 660
catctgtgta caccggagtg tttgtagtca gcctcccact aaacatcatg gccatcgttg 720
tgttcatcct gaaaatgaag gtcaagaagc cggcggtggt gtacatgctg cacctggcca 780
cggcagatgt gctgtttgtg tctgtgctcc cctttaagat cagctattac ttttccggca 840
gtgattggca gtttgggtct gaattgtgtc gcttcgtcac tgcagcattt tactgtaaca 900
tgtacgcctc tatcttgctc atgacagtca taagcattga ccggtttctg gctgtggtgt 960
atcccatgca gtccctctcc tggcgtactc tgggaagggc ttccttcact tgtctggcca 1020
tctgggcttt ggccatcgca ggggtagtgc ctctcgtcct caaggagcaa accatccagg 1080
tgcccgggct caacatcact acctgtcatg atgtgctcaa tgaaaccctg ctcgaaggct 1140
actatgccta ctacttctca gccttctctg ctgtcttctt ttttgtgccg ctgatcattt 1200
ccacggtctg ttatgtgtct atcattcgat gtcttagctc ttccgcagtt gccaaccgca 1260
gcaagaagtc ccgggctttg ttcctgtcag ctgctgtttt ctgcatcttc atcatttgct 1320
tcggacccac aaacgtcctc ctgattgcgc attactcatt cctttctcac acttccacca 1380
cagaggctgc ctactttgcc tacctcctct gtgtctgtgt cagcagcata agctcgtgca 1440
tcgacccccct aatttactat tacgcttcct ctgagtgcca gaggtacgtc tacagtatct 1500
tatgctgcaa agaaagttcc gatcccagca gttataacag cagtgggcag ttgatggcaa 1560
```

gtaaaatgga tacctgctct agtaacctga ataacagcat atacaaaaag ctgttaactt 1620
aggaaaaggg actgctggga ggttaaaaag aaaagtttat aaaagtgaat aacctgagga 1680
ttctattagt ccccacccaa actttattga ttcacctcct aaaacaacag atgtacgact 1740
tgcatacctg ctttttatgg gagctgtcaa gcatgtattt ttgtcaatta ccagaaagat 1800
aacaggacga gatgacggtg ttattccaag ggaatattgc caatgctaca gtaataaatg 1860
aatgtcactt ctggatatag ctaggtgaca tatacatact tacatgtgtg tatatgtaga 1920
tgtatgcaca cacatatatt atttgcagtg cagtatagaa taggcacttt aaaacactct 1980
ttccccgcac cccagcaatt atgaaaataa tctctgattc cctgatttaa tatgcaaagt 2040
ctaggttggt agagtttagc cctgaacatt tcatggtgtt catcaacagt gagagactcc 2100
atagtttggg cttgtaccac ttttgcaaat aagtgtattt tgaaattgtt tgacggcaag 2160
gtttaagtta ttaagaggta agacttagta ctatctgtgc gtagaagttc tagtgttttc 2220
aattttaaac atatccaagt ttgaattcct aaaattatgg aaacagatga aaagcctctg 2280
ttttgatatg ggtagtattt tttacatttt acacactgta cacataagcc aaaactgagc 2340
ataagtcctc tagtgaatgt aggctggctt tcagagtagg ctattcctga gagctgcatg 2400
tgtccgcccc cgatggagga ctccaggcag cagacacatg ccagggccat gtcagacaca 2460
gattggccag aaaccttcct gctgagcctc acagcagtga gactggggcc actacatttg 2520
ctccatcctc ctgggattgg ctgtgaactg atcatgttta tgagaaactg gcaaagcaga 2580
atgtgatatc ctaggaggta atgaccatga aagacttctc tacccatctt aaaaacaacg 2640
aaagaaggca tggacttctg gatgcccatc cactgggtgt aaacacatct agtagttgtt 2700
ctgaaatgtc agttctgata tggaagcacc cattatgcgc tgtggccact ccaataggtg 2760
ctgagtgtac agagtggaat aagacagaga cctgccctca agagcaaagt agatcatgca 2820
tagagtgtga tgtatgtgta ataaatatgt ttcacacaaa caaggcctgt cagctaaaga 2880
agtttgaaca tttgggttac tatttcttgt ggttataact taatgaaaac aatgcagtac 2940
aggacatata tttttaaaa taagtctgat ttaattgggc actatttatt tacaaatgtt 3000
ttgctcaata gattgctcaa atcaggtttt cttttaagaa tcaatcatgt cagtctgctt 3060
agaaataaca gaagaaaata gaattgacat tgaaatctag gaaaattatt ctataatttc 3120
catttactta agacttaatg agactttaaa agcatttttt aacctcctaa gtatcaagta 3180
tagaaaatct tcatggaatt cacaaagtaa tttggaaatt aggttgaaac atatctctta 3240
tcttacgaaa aaatggtagc attttaaaca aaatagaaag ttgcaaggca aatgtttatt 3300
taaaagagca ggccaggcgc ggtggctcac gcctgtaatc ccagcacttt gggaggctga 3360
ggcgggtgga tcacgaggtc aggagatcga gaccatcctg gctaacacgg tgaaacccgt 3420
ctctactaaa aatgcaaaaa aaattagccg ggcgtggtgg caggcacctg tagtcccagc 3480
tactcgggag gctgaggcag gagactggcg tgaacccagg aggcggacct tgtagtgagc 3540
cgagatcgcg ccactgtgct ccagcctggg caacagagca agactccatc tc 3592

<210> 2
<211> 3592
<212> DNA
<213> Homo sapiens

<400> 2

```
ggcgggggc gcacagagcc agaggggctt gcgagcggcg gctgagggac cgcggggagg 60
gggcgccgag cggctccagc gcagagactc tcactgcacg ccggaggccc cttcctcgct 120
ccgcccgcgc gaccgcgcgc cccagtcccg ccccgccccg ctaaccgccc cagacacagc 180
gctcgccgag ggtcgcttgg accctgatct tacccgtggg caccctgcgc tctgcctgcc 240
gcgaagaccg gctccccgac ccgcagaagt caggagagag ggtgaagcgg agcagcccga 300
ggcggggcag cctcccggag cagcgccgcg cagagcccgg gacaatgggg ccgcggcggc 360
```

tgctgctggt ggccgcctgc ttcagtctgt gcggcccgct gttgtctgcc cgcacccggg 420
cccgcaggcc agaatcaaaa gcaacaaatg ccaccttaga tccccggtca tttcttctca 480
ggaaccccaa tgataaatat gaaccatttt gggaggatga ggagaaaaat gaaagtgggt 540
taactgaata cagattagtc tccatcaata aaagcagtcc tcttcaaaaa caacttcctg 600
cattcatctc agaagatgcc tccggatatt tgaccagctc ctggctgaca ctctttgtcc 660
catctgtgta caccggagtg tttgtagtca gcctcccact aaacatcatg gccatcgttg 720
tgttcatcct gaaaatgaag gtcaagaagc cggcggtggt gtacatgctg cacctggcca 780
cggcagatgt gctgtttgtg tctgtgctcc cctttaagat cagctattac ttttccggca 840
gtgattggca gtttgggtct gaattgtgtc gcttcgtcac tgcagcattt tactgtaaca 900
tgtacgcctc tatcttgctc atgacagtca taagcattga ccggtttctg gctgtggtgt 960
atcccatgca gtccctctcc tggcgtactc tgggaagggc ttccttcact tgtctggcca 1020
tctgggcttt ggccatcgca ggggtagtgc ctctcgtcct caaggagcaa accatccagg 1080
tgcccgggct caacatcact acctgtcatg atgtgctcaa tgaaaccctg ctcgaaggct 1140
actatgccta ctacttctca gccttctctg ctgtcttctt ttttgtgccg ctgatcattt 1200
ccacggtctg ttatgtgtct atcattcgat gtcttagctc ttccgcagtt gccaaccgca 1260
gcaagaagtc ccgggctttg ttcctgtcag ctgctgtttt ctgcatcttc atcatttgct 1320
tcggacccac aaacgtcctc ctgattgcgc attactcatt cctttctcac acttccacca 1380
cagaggctgc ctactttgcc tacctcctct gtgtctgtgt cagcagcata agctcgtgca 1440
tcgacccccct aatttactat tacgcttcct ctgagtgcca gaggtacgtc tacagtatct 1500
tatgctgcaa agaaagttcc gatcccagca gttataacag cagtgggcag ttgatggcaa 1560
gtaaaatgga tacctgctct agtaacctga ataacagcat atacaaaaag ctgttaactt 1620
aggaaaaggg actgctggga ggttaaaaag aaaagtttat aaaagtgaat aacctgagga 1680
ttctattagt ccccacccaa actttattga ttcacctcct aaaacaacag atgtacgact 1740
tgcatacctg ctttttatgg gagctgtcaa gcatgtattt ttgtcaatta ccagaaagat 1800
aacaggacga gatgacggtg ttattccaag ggaatattgc caatgctaca gtaataaatg 1860
aatgtcactt ctggatatag ctaggtgaca tatacatact tacatgtgtg tatatgtaga 1920
tgtatgcaca cacatatatt atttgcagtg cagtatagaa taggcacttt aaaacactct 1980
ttccccgcac cccagcaatt atgaaaataa tctctgattc cctgatttaa tatgcaaagt 2040
ctaggttggt agagtttagc cctgaacatt tcatggtgtt catcaacagt gagagactcc 2100
atagtttggg cttgtaccac ttttgcaaat aagtgtattt tgaaattgtt tgacggcaag 2160
gtttaagtta ttaagaggta agacttagta ctatctgtgc gtagaagttc tagtgttttc 2220
aattttaaac atatccaagt ttgaattcct aaaattatgg aaacagatga aaagcctctg 2280
ttttgatatg ggtagtattt tttacatttt acacactgta cacataagcc aaaactgagc 2340
ataagtcctc tagtgaatgt aggctggctt tcagagtagg ctattcctga gagctgcatg 2400
tgtccgcccc cgatggagga ctccaggcag cagacacatg ccagggccat gtcagacaca 2460
gattggccag aaaccttcct gctgagcctc acagcagtga gactggggcc actacatttg 2520
ctccatcctc ctgggattgg ctgtgaactg atcatgttta tgagaaactg gcaaagcaga 2580
atgtgatatc ctaggaggta atgaccatga aagacttctc tacccatctt aaaaacaacg 2640
aaagaaggca tggacttctg gatgcccatc cactgggtgt aaacacatct agtagttgtt 2700
ctgaaatgtc agttctgata tggaagcacc cattatgcgc tgtggccact ccaataggtg 2760
ctgagtgtac agagtggaat aagacagaga cctgccctca agagcaaagt agatcatgca 2820
tagagtgtga tgtatgtgta ataaatatgt ttcacacaaa caaggcctgt cagctaaaga 2880
agtttgaaca tttgggttac tatttcttgt ggttataact taatgaaaac aatgcagtac 2940
aggacatata tttttaaaa taagtctgat ttaattgggc actatttatt tacaaatgtt 3000
ttgctcaata gattgctcaa atcaggtttt cttttaagaa tcaatcatgt cagtctgctt 3060
agaaataaca gaagaaaata gaattgacac tgaaatctag gaaaattatt ctataatttc 3120
catttactta agacttaatg agactttaaa agcatttttt aacctcctaa gtatcaagta 3180
tagaaaatct tcatggaatt cacaaagtaa tttggaaatt aggttgaaac atatctctta 3240

```
tcttacgaaa aaatggtagc attttaaaca aaatagaaag ttgcaaggca aatgtttatt 3300
taaaagagca ggccaggcgc ggtggctcac gcctgtaatc ccagcacttt gggaggctga 3360
ggcgggtgga tcacgaggtc aggagatcga gaccatcctg gctaacacgg tgaaacccgt 3420
ctctactaaa aatgcaaaaa aaattagccg ggcgtggtgg caggcacctg tagtcccagc 3480
tactcgggag gctgaggcag gagactggcg tgaacccagg aggcggacct tgtagtgagc 3540
cgagatcgcg ccactgtgct ccagcctggg caacagagca agactccatc tc        3592
```

<210> 3
<211> 3592
<212> DNA
<213> Homo sapiens

<400> 3

ggcgggggc gcacagagcc agaggggctt gcgagcggcg gctgagggac cgcggggagg 60
gggcgccgag cggctccagc gcagagactc tcactgcacg ccggaggccc cttcctcgct 120
ccgcccgcgc gaccgcgcgc cccagtcccg ccccgccccg ctaaccgccc cagacacagc 180
gctcgccgag ggtcgcttgg accctgatct tacccgtggg caccctgcgc tctgcctgcc 240
gcgaagaccg gctccccgac ccgcagaagt caggagagag ggtgaagcgg agcagcccga 300
ggcggggcag cctcccggag cagcgccgcg cagagcccgg gacaatgggg ccgcggcggc 360
tgctgctggt ggccgcctgc ttcagtctgt gcggcccgct gttgtctgcc cgcacccggg 420
cccgcaggcc agaatcaaaa gcaacaaatg ccaccttaga tccccggtca tttcttctca 480
ggaaccccaa tgataaatat gaaccatttt gggaggatga ggagaaaaat gaaagtgggt 540
taactgaata cagattagtc tccatcaata aaagcagtcc tcttcaaaaa caacttcctg 600
cattcatctc agaagatgcc tccggatatt tgaccagctc ctggctgaca ctctttgtcc 660
catctgtgta caccggagtg tttgtagtca gcctcccact aaacatcatg gccatcgttg 720
tgttcatcct gaaaatgaag gtcaagaagc cggcggtggt gtacatgctg cacctggcca 780
cggcagatgt gctgtttgtg tctgtgctcc cctttaagat cagctattac ttttccggca 840
gtgattggca gtttgggtct gaattgtgtc gcttcgtcac tgcagcattt tactgtaaca 900
tgtacgcctc tatcttgctc atgacagtca taagcattga ccggtttctg gctgtggtgt 960
atcccatgca gtccctctcc tggcgtactc tgggaagggc ttccttcact tgtctggcca 1020
tctgggcttt ggccatcgca ggggtagtgc ctctcgtcct caaggagcaa accatccagg 1080
tgcccgggct caacatcact acctgtcatg atgtgctcaa tgaaaccctg ctcgaaggct 1140
actatgccta ctacttctca gccttctctg ctgtcttctt ttttgtgccg ctgatcattt 1200
ccacggtctg ttatgtgtct atcattcgat gtcttagctc ttccgcagtt gccaaccgca 1260
gcaagaagtc ccgggctttg ttcctgtcag ctgctgtttt ctgcatcttc atcatttgct 1320
tcggacccac aaacgtcctc ctgattgcgc attactcatt cctttctcac acttccacca 1380
cagaggctgc ctactttgcc tacctcctct gtgtctgtgt cagcagcata agctcgtgca 1440
tcgacccccct aatttactat tacgcttcct ctgagtgcca gaggtacgtc tacagtatct 1500
tatgctgcaa agaaagttcc gatcccagca gttataacag cagtgggcag ttgatggcaa 1560
gtaaaatgga tacctgctct agtaacctga ataacagcat atacaaaaag ctgttaactt 1620
aggaaaaggg actgctggga ggttaaaaag aaaagtttat aaaagtgaat aacctgagga 1680
ttctattagt ccccacccaa actttattga ttcacctcct aaaacaacag atgtacgact 1740
tgcatacctg ctttttatgg gagctgtcaa gcatgtattt ttgtcaatta ccagaaagat 1800
aacaggacga gatgacggtg ttattccaag ggaatattgc caatgctaca gtaataaatg 1860
aatgtcactt ctggatatag ctaggtgaca tatacatact tacatgtgtg tatatgtaga 1920
tgtatgcaca cacatatatt atttgcagtg cagtatagaa taggcacttt aaaacactct 1980
ttccccgcac cccagcaatt atgaaaataa tctctgattc cctgatttaa tatgcaaagt 2040

ctaggttggt agagtttagc cctgaacatt tcatggtgtt catcaacagt gagagactcc 2100
atagtttggg cttgtaccac ttttgcaaat aagtgtattt tgaaattgtt tgacggcaag 2160
gtttaagtta ttaagaggta agacttagta ctatctgtgc gtagaagttc tagtgttttc 2220
aattttaaac atatccaagt ttgaattcct aaaattatgg aaacagatga aaagcctctg 2280
ttttgatatg ggtagtattt tttacatttt acacactgta cacataagcc aaaactgagc 2340
ataagtcctc tagtgaatgt aggctggctt tcagagtagg ctattcctga gagctgcatg 2400
tgtccgcccc cgatggagga ctccaggcag cagacacatg ccagggccat gtcagacaca 2460
gattggccag aaaccttcct gctgagcctc acagcagtga gactggggcc actacatttg 2520
ctccatcctc ctgggattgg ctgtgaactg atcatgttta tgagaaactg gcaaagcaga 2580
atgtgatatc ctaggaggta atgaccatga aagacttctc tacccatctt aaaaacaacg 2640
aaagaaggca tggacttctg gatgcccatc cactgggtgt aaacacatct agtagttgtt 2700
ctgaaatgtc agttctgata tggaagcacc cattatgcgc tgtggccact ccaataggtg 2760
ctgagtgtac agagtggaat aagacagaga cctgccctca agagcaaagt agatcatgca 2820
tagagtgtga tgtatgtgta ataaatatgt ttcacacaaa caaggcctgt cagctaaaga 2880
agtttgaaca tttgggttac tatttcttgt ggttataact taatgaaaac aatgcagtac 2940
aggacatata ttttttaaaa taagtctgat ttaattgggc actatttatt tacaaatgtt 3000
ttgctcaata gattgctcaa atcaggtttt cttttaagaa tcaatcatgt cagtctgctt 3060
agaaataaca gaagaaaata gaattgacat tgaaatctag gaaaattatt ctataatttc 3120
catttactta agacttaatg agactttaaa agcatttttt aacctcctaa gtatcaagta 3180
tagaaaatct tcatggaatt cacaaagtaa tttggaaatt aggttgaaac atatctctta 3240
tcttacgaaa aaatggtagc attttaaaca aaatagaaag ttgcaaggca aatgtttatt 3300
taaaagagca ggccaggcgc ggtggctccc gcctgtaatc ccagcacttt gggaggctga 3360
ggcgggtgga tcacgaggtc aggagatcga gaccatcctg gctaacacgg tgaaaccccgt 3420
ctctactaaa aatgcaaaaa aaattagccg ggcgtggtgg caggcacctg tagtcccagc 3480
tactcgggag gctgaggcag gagactggcg tgaacccagg aggcggacct tgtagtgagc 3540
cgagatcgcg ccactgtgct ccagcctggg caacagagca agactccatc tc 3592

<210> 4
<211> 3592
<212> DNA
<213> Homo sapiens

<400> 4

ggcgggggc gcacagagcc agaggggctt gcgagcggcg gctgagggac cgcggggagg 60
gggcgccgag cggctccagc gcagagactc tcactgcacg ccggaggccc cttcctcgct 120
ccgcccgcgc gaccgcgcgc cccagtcccg ccccgccccg ctaaccgccc cagacacagc 180
gctcgccgag ggtcgcttgg accctgatct tacccgtggg caccctgcgc tctgcctgcc 240
gcgaagaccg gctccccgac ccgcagaagt caggagagag ggtgaagcgg agcagcccga 300
ggcggggcag cctcccggag cagcgccgcg cagagcccgg gacaatgggg ccgcggcggc 360
tgctgctggt ggccgcctgc ttcagtctgt gcggcccgct gttgtctgcc cgcacccggg 420
cccgcaggcc agaatcaaaa gcaacaaatg ccaccttaga tccccggtca tttcttctca 480
ggaaccccaa tgataaatat gaaccatttt gggaggatga ggagaaaaat gaaagtgggt 540
taactgaata cagattagtc tccatcaata aaagcagtcc tcttcaaaaa caacttcctg 600
cattcatctc agaagatgcc tccggatatt tgaccagctc ctggctgaca ctctttgtcc 660
catctgtgta caccggagtg tttgtagtca gcctcccact aaacatcatg gccatcgttg 720
tgttcatcct gaaaatgaag gtcaagaagc cggcggtggt gtacatgctg cacctggcca 780
cggcagatgt gctgtttgtg tctgtgctcc cctttaagat cagctattac ttttccggca 840

```
gtgattggca gtttgggtct gaattgtgtc gcttcgtcac tgcagcattt tactgtaaca 900
tgtacgcctc tatcttgctc atgacagtca taagcattga ccggtttctg gctgtggtgt 960
atcccatgca gtccctctcc tggcgtactc tgggaagggc ttccttcact tgtctggcca 1020
tctgggcttt ggccatcgca ggggtagtgc ctctcgtcct caaggagcaa accatccagg 1080
tgcccgggct caacatcact acctgtcatg atgtgctcaa tgaaaccctg ctcgaaggct 1140
actatgccta ctacttctca gccttctctg ctgtcttctt ttttgtgccg ctgatcattt 1200
ccacggtctg ttatgtgtct atcattcgat gtcttagctc ttccgcagtt gccaaccgca 1260
gcaagaagtc ccgggctttg ttcctgtcag ctgctgtttt ctgcatcttc atcatttgct 1320
tcggacccac aaacgtcctc ctgattgcgc attactcatt cctttctcac acttccacca 1380
cagaggctgc ctactttgcc tacctcctct gtgtctgtgt cagcagcata agctcgtgca 1440
tcgacccccct aatttactat tacgcttcct ctgagtgcca gaggtacgtc tacagtatct 1500
tatgctgcaa agaaagttcc gatcccagca gttataacag cagtgggcag ttgatggcaa 1560
gtaaaatgga tacctgctct agtaacctga ataacagcat atacaaaaag ctgttaactt 1620
aggaaaaggg actgctggga ggttaaaaag aaaagtttat aaaagtgaat aacctgagga 1680
ttctattagt ccccacccaa actttattga ttcacctcct aaaacaacag atgtacgact 1740
tgcatacctg ctttttatgg gagctgtcaa gcatgtattt ttgtcaatta ccagaaagat 1800
aacaggacga gatgacggtg ttattccaag ggaatattgc caatgctaca gtaataaatg 1860
aatgtcactt ctggatatag ctaggtgaca tatacatact tacatgtgtg tatatgtaga 1920
tgtatgcaca cacatatatt atttgcagtg cagtatagaa taggcacttt aaaacactct 1980
ttccccgcac cccagcaatt atgaaaataa tctctgattc cctgatttaa tatgcaaagt 2040
ctaggttggt agagtttagc cctgaacatt tcatggtgtt catcaacagt gagagactcc 2100
atagtttggg cttgtaccac ttttgcaaat aagtgtattt tgaaattgtt tgacggcaag 2160
gtttaagtta ttaagaggta agacttagta ctatctgtgc gtagaagttc tagtgttttc 2220
aattttaaac atatccaagt ttgaattcct aaaattatgg aaacagatga aaagcctctg 2280
ttttgatatg ggtagtattt tttacatttt acacactgta cacataagcc aaaactgagc 2340
ataagtcctc tagtgaatgt aggctggctt tcagagtagg ctattcctga gagctgcatg 2400
tgtccgcccc cgatggagga ctccaggcag cagacacatg ccagggccat gtcagacaca 2460
gattggccag aaaccttcct gctgagcctc acagcagtga gactggggcc actacatttg 2520
ctccatcctc ctgggattgg ctgtgaactg atcatgttta tgagaaactg gcaaagcaga 2580
atgtgatatc ctaggaggta atgaccatga aagacttctc tacccatctt aaaaacaacg 2640
aaagaaggca tggacttctg gatgcccatc cactgggtgt aaacacatct agtagttgtt 2700
ctgaaatgtc agttctgata tggaagcacc cattatgcgc tgtggccact ccaataggtg 2760
ctgagtgtac agagtggaat aagacagaga cctgccctca agagcaaagt agatcatgca 2820
tagagtgtga tgtatgtgta ataaatatgt ttcacacaaa caaggcctgt cagctaaaga 2880
agtttgaaca tttgggttac tatttcttgt ggttataact taatgaaaac aatgcagtac 2940
aggacatata ttttttaaaa taagtctgat ttaattgggc actatttatt tacaaatgtt 3000
ttgctcaata gattgctcaa atcaggtttt cttttaagaa tcaatcatgt cagtctgctt 3060
agaaataaca gaagaaaata gaattgacac tgaaatctag gaaaattatt ctataatttc 3120
catttactta agacttaatg agactttaaa agcatttttt aacctcctaa gtatcaagta 3180
tagaaaatct tcatggaatt cacaaagtaa tttggaaatt aggttgaaac atatctctta 3240
tcttacgaaa aaatggtagc attttaaaca aaatagaaag ttgcaaggca aatgtttatt 3300
taaaagagca ggccaggcgc ggtggctccc gcctgtaatc ccagcacttt gggaggctga 3360
ggcgggtgga tcacgaggtc aggagatcga gaccatcctg gctaacacgg tgaaacccgt 3420
ctctactaaa aatgcaaaaa aaattagccg ggcgtggtgg caggcacctg tagtcccagc 3480
tactcgggag gctgaggcag gagactggcg tgaacccagg aggcggacct tgtagtgagc 3540
cgagatcgcg ccactgtgct ccagcctggg caacagagca agactccatc tc         3592
```

<210> 5
<211> 939
<212> DNA
<213> Homo sapiens

<400> 5

acagagtgga ataagacaga gacctgccct caagagcaaa gtagatcatg catagagtgt 60
gatgtatgtg taataaatat gtttcacaca aacaaggcct gtcagctaaa gaagtttgaa 120
catttgggtt actatttctt gtggttataa cttaatgaaa acaatgcagt acaggacata 180
tattttttaa aataagtctg atttaattgg gcactattta tttacaaatg ttttgctcaa 240
tagattgctc aaatcaggtt ttcttttaag aatcaatcat gtcagtctgc ttagaaataa 300
cagaagaaaa tagaattgac attgaaatct aggaaaatta ttctataatt tccatttact 360
taagacttaa tgagacttta aaagcatttt ttaacctcct aagtatcaag tatagaaaat 420
cttcatggaa ttcacaaagt aatttggaaa ttaggttgaa acatatctct tatcttacga 480
aaaaatggta gcattttaaa caaaatagaa agttgcaagg caaatgttta tttaaaagag 540
caggccaggc gcggtggctc acgcctgtaa tcccagcact ttgggaggct gaggcgggtg 600
gatcacgagg tcaggagatc gagaccatcc tggctaacac ggtgaaaccc gtctctacta 660
aaaatgcaaa aaaaattagc cgggcgtggt ggcaggcacc tgtagtccca gctactcggg 720
aggctgaggc aggagactgg cgtgaaccca ggaggcggac cttgtagtga gccgagatcg 780
cgccactgtg ctccagcctg ggcaacagag caagactcca tctcaaaaaa taaaaataaa 840
taaaaaataa aaaaataaaa gagcaaacta tttccaaata ccatagaata acttacataa 900
aagtaatata actgtattgt aagtagaagc tagcactgg 939

<210> 6
<211> 939
<212> DNA
<213> Homo sapiens

<400> 6

```
acagagtgga ataagacaga gacctgccct caagagcaaa gtagatcatg catagagtgt 60
gatgtatgtg taataaatat gtttcacaca aacaaggcct gtcagctaaa gaagtttgaa 120
catttgggtt actatttctt gtggttataa cttaatgaaa acaatgcagt acaggacata 180
tatttttaa aataagtctg atttaattgg gcactattta tttacaaatg ttttgctcaa 240
tagattgctc aaatcaggtt ttcttttaag aatcaatcat gtcagtctgc ttagaaataa 300
cagaagaaaa tagaattgac actgaaatct aggaaaatta ttctataatt tccatttact 360
taagacttaa tgagacttta aaagcatttt ttaacctcct aagtatcaag tatagaaaat 420
cttcatggaa ttcacaaagt aatttggaaa ttaggttgaa acatatctct tatcttacga 480
aaaaatggta gcattttaaa caaaatagaa agttgcaagg caaatgttta tttaaaagag 540
caggccaggc gcggtggctc acgcctgtaa tcccagcact ttgggaggct gaggcgggtg 600
gatcacgagg tcaggagatc gagaccatcc tggctaacac ggtgaaaccc gtctctacta 660
aaaatgcaaa aaaaattagc cgggcgtggt ggcaggcacc tgtagtccca gctactcggg 720
aggctgaggc aggagactgg cgtgaaccca ggaggcggac cttgtagtga gccgagatcg 780
cgccactgtg ctccagcctg ggcaacagag caagactcca tctcaaaaaa taaaaataaa 840
taaaaaataa aaaaataaaa gagcaaacta tttccaaata ccatagaata acttacataa 900
aagtaatata actgtattgt aagtagaagc tagcactgg                        939
```

<210> 7
<211> 939
<212> DNA
<213> Homo sapiens

<400> 7

```
acagagtgga ataagacaga gacctgccct caagagcaaa gtagatcatg catagagtgt 60
gatgtatgtg taataaatat gtttcacaca aacaaggcct gtcagctaaa gaagtttgaa 120
catttgggtt actatttctt gtggttataa cttaatgaaa acaatgcagt acaggacata 180
tatttttaa aataagtctg atttaattgg gcactattta tttacaaatg ttttgctcaa 240
tagattgctc aaatcaggtt ttcttttaag aatcaatcat gtcagtctgc ttagaaataa 300
cagaagaaaa tagaattgac attgaaatct aggaaaatta ttctataatt tccatttact 360
taagacttaa tgagacttta aaagcatttt ttaacctcct aagtatcaag tatagaaaat 420
cttcatggaa ttcacaaagt aatttggaaa ttaggttgaa acatatctct tatcttacga 480
aaaaatggta gcattttaaa caaaatagaa agttgcaagg caaatgttta tttaaaagag 540
caggccaggc gcggtggctc ccgcctgtaa tcccagcact ttgggaggct gaggcgggtg 600
gatcacgagg tcaggagatc gagaccatcc tggctaacac ggtgaaaccc gtctctacta 660
aaaatgcaaa aaaaattagc cgggcgtggt ggcaggcacc tgtagtccca gctactcggg 720
aggctgaggc aggagactgg cgtgaaccca ggaggcggac cttgtagtga gccgagatcg 780
cgccactgtg ctccagcctg ggcaacagag caagactcca tctcaaaaaa taaaaataaa 840
taaaaaataa aaaaataaaa gagcaaacta tttccaaata ccatagaata acttacataa 900
aagtaatata actgtattgt aagtagaagc tagcactgg                        939
```

<210> 8
<211> 939
<212> DNA

<213> Homo sapiens

<400> 8

acagagtgga ataagacaga gacctgccct caagagcaaa gtagatcatg catagagtgt 60
gatgtatgtg taataaatat gtttcacaca aacaaggcct gtcagctaaa gaagtttgaa 120
catttgggtt actatttctt gtggttataa cttaatgaaa acaatgcagt acaggacata 180
tatttttttaa aataagtctg atttaattgg gcactattta tttacaaatg ttttgctcaa 240
tagattgctc aaatcaggtt ttcttttaag aatcaatcat gtcagtctgc ttagaaataa 300
cagaagaaaa tagaattgac actgaaatct aggaaaatta ttctataatt tccatttact 360
taagacttaa tgagacttta aaagcatttt ttaacctcct aagtatcaag tatagaaaat 420
cttcatggaa ttcacaaagt aatttggaaa ttaggttgaa acatatctct tatcttacga 480
aaaaatggta gcattttaaa caaaatagaa agttgcaagg caaatgttta tttaaaagag 540
caggccaggc gcggtggctc ccgcctgtaa tcccagcact ttgggaggct gaggcgggtg 600
gatcacgagg tcaggagatc gagaccatcc tggctaacac ggtgaaaccc gtctctacta 660
aaaatgcaaa aaaaattagc cgggcgtggt ggcaggcacc tgtagtccca gctactcggg 720
aggctgaggc aggagactgg cgtgaaccca ggaggcggac cttgtagtga gccgagatcg 780
cgccactgtg ctccagcctg ggcaacagag caagactcca tctcaaaaaa taaaaataaa 840
taaaaaataa aaaaataaaa gagcaaacta tttccaaata ccatagaata acttacataa 900
aagtaatata actgtattgt aagtagaagc tagcactgg 939

<210> 9
<211> 20
<212> DNA
<213> Homo sapiens

<400> 9
ggcggggggc gcacagagcc 20

<210> 10
<211> 22
<212> DNA
<213> Homo sapiens

<400> 10
gagatggagt cttgctctgt tg 22

<210> 11
<211> 21
<212> DNA
<213> Homo sapiens

<400> 11
acagagtgga ataagacaga g 21

<210> 12
<211> 21
<212> DNA
<213> Homo sapiens

<400> 12
ccagtgctag cttctactta c 21

<210> 13
<211> 425
<212> PRT
<213> Homo sapiens

<400> 13

Met Gly Pro Arg Arg Leu Leu Leu Val Ala Ala Cys Phe Ser Leu Cys
1 5 10 15

Gly Pro Leu Leu Ser Ala Arg Thr Arg Ala Arg Arg Pro Glu Ser Lys
20 25 30

```
Ala Thr Asn Ala Thr Leu Asp Pro Arg Ser Phe Leu Leu Arg Asn Pro
        35                  40                  45

Asn Asp Lys Tyr Glu Pro Phe Trp Glu Asp Glu Glu Lys Asn Glu Ser
    50                  55                  60

Gly Leu Thr Glu Tyr Arg Leu Val Ser Ile Asn Lys Ser Ser Pro Leu
65                  70                  75                  80

Gln Lys Gln Leu Pro Ala Phe Ile Ser Glu Asp Ala Ser Gly Tyr Leu
                85                  90                  95

Thr Ser Ser Trp Leu Thr Leu Phe Val Pro Ser Val Tyr Thr Gly Val
            100                 105                 110

Phe Val Val Ser Leu Pro Leu Asn Ile Met Ala Ile Val Val Phe Ile
        115                 120                 125

Leu Lys Met Lys Val Lys Lys Pro Ala Val Val Tyr Met Leu His Leu
    130                 135                 140

Ala Thr Ala Asp Val Leu Phe Val Ser Val Leu Pro Phe Lys Ile Ser
145                 150                 155                 160

Tyr Tyr Phe Ser Gly Ser Asp Trp Gln Phe Gly Ser Glu Leu Cys Arg
                165                 170                 175

Phe Val Thr Ala Ala Phe Tyr Cys Asn Met Tyr Ala Ser Ile Leu Leu
            180                 185                 190

Met Thr Val Ile Ser Ile Asp Arg Phe Leu Ala Val Val Tyr Pro Met
        195                 200                 205

Gln Ser Leu Ser Trp Arg Thr Leu Gly Arg Ala Ser Phe Thr Cys Leu
    210                 215                 220

Ala Ile Trp Ala Leu Ala Ile Ala Gly Val Val Pro Leu Val Leu Lys
225                 230                 235                 240

Glu Gln Thr Ile Gln Val Pro Gly Leu Asn Ile Thr Thr Cys His Asp
                245                 250                 255

Val Leu Asn Glu Thr Leu Leu Glu Gly Tyr Tyr Ala Tyr Tyr Phe Ser
            260                 265                 270

Ala Phe Ser Ala Val Phe Phe Phe Val Pro Leu Ile Ile Ser Thr Val
        275                 280                 285
```

```
Cys Tyr Val Ser Ile Ile Arg Cys Leu Ser Ser Ser Ala Val Ala Asn
    290                 295                 300

Arg Ser Lys Lys Ser Arg Ala Leu Phe Leu Ser Ala Ala Val Phe Cys
305                 310                 315                 320

Ile Phe Ile Ile Cys Phe Gly Pro Thr Asn Val Leu Leu Ile Ala His
                325                 330                 335

Tyr Ser Phe Leu Ser His Thr Ser Thr Thr Glu Ala Ala Tyr Phe Ala
            340                 345                 350

Tyr Leu Leu Cys Val Cys Val Ser Ser Ile Ser Ser Cys Ile Asp Pro
        355                 360                 365

Leu Ile Tyr Tyr Tyr Ala Ser Ser Glu Cys Gln Arg Tyr Val Tyr Ser
    370                 375                 380

Ile Leu Cys Cys Lys Glu Ser Ser Asp Pro Ser Ser Tyr Asn Ser Ser
385                 390                 395                 400

Gly Gln Leu Met Ala Ser Lys Met Asp Thr Cys Ser Ser Asn Leu Asn
                405                 410                 415

Asn Ser Ile Tyr Lys Lys Leu Leu Thr
            420                 425
```